(19) Europäisches Patentamt
European Patent Office
Office européen des brevets

(11) EP 3 039 733 B1

(12) EUROPEAN PATENT SPECIFICATION

(45) Date of publication and mention of the grant of the patent:
**21.11.2018 Bulletin 2018/47**

(21) Application number: **14840167.2**

(22) Date of filing: **29.08.2014**

(51) Int Cl.:
***H01M 4/02*** *(2006.01)* ***G01N 27/327*** *(2006.01)*
***G01N 33/487*** *(2006.01)*

(86) International application number:
**PCT/US2014/053486**

(87) International publication number:
**WO 2015/031798 (05.03.2015 Gazette 2015/09)**

(54) **MATRIX-ENHANCED ELECTROCHEMICAL DETECTOR FOR PATHOGENIC BACTERIA**

MATRIXERWEITERTER ELEKTROCHEMISCHER DETEKTOR PATHOGENER BAKTERIEN

DÉTECTEUR ÉLECTROCHIMIQUE OPTIMISÉ PAR UNE MATRICE POUR LES BACTÉRIES PATHOGÈNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.08.2013 US 201361871373 P**

(43) Date of publication of application:
**06.07.2016 Bulletin 2016/27**

(73) Proprietor: **Northeastern University**
**Boston, MA 02115 (US)**

(72) Inventors:
- **GOLUCH, Edgar D.**
  **Somerville Massachusetts 02144 (US)**
- **SISMAET, Hunter J.**
  **Boston, MA 02135 (US)**
- **WEBSTER, Thaddaeus A.**
  **Exeter, New Hampshire 03833 (US)**

(74) Representative: **Høiberg P/S**
**Adelgade 12**
**1304 Copenhagen K (DK)**

(56) References cited:
**WO-A1-2005/112151**

- **Thaddaeus A Webster: "Detection of Quorum Sensing Molecules in Nanofluidic Detection Devices", NORTHEASTERN UNIVERSITY, 23 July 2011 (2011-07-23), XP055182376, Retrieved from the Internet: URL:http://nuweb9.neu.edu/chegsc/wp-content/uploads/Webster-Abstract-03-11-111.pdf [retrieved on 2015-04-13]**
- **THADDAEUS A. WEBSTER ET AL: "Electrochemical detection of pyocyanin in nanochannels with integrated palladium hydride reference electrodes", LAB ON A CHIP, vol. 12, no. 24, 1 January 2012 (2012-01-01), page 5195, XP055257916, ISSN: 1473-0197, DOI: 10.1039/c2lc40650k**
- **WEBSTER ET AL.: 'Amperometric Detection of Pyocyanin in Nanofluidic Channels' NANO LIFE vol. 3, no. 1, 2013, page 1340011, XP055321110**
- **GUO ET AL.: 'Analysis of underivatized amino acids by capillary electrophoresis using constant potential amperometric detection' ELECTROPHORESIS vol. 16, 1995, pages 493 - 491, XP055321111**
- **SILVESTRI ET AL.: 'Micromachined flow sensors in biomedical applications' MICROMACHINES vol. 3, 2012, pages 225 - 243, XP055321112**
- **GODINO ET AL.: 'Fabricating electrodes for amperometric detection in hybrid paper/polymer lab-on- a-chip devices' LAB CHIP 2012, pages 3281 - 3284, XP055237202**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **ACUMEDIA: 'TRYPTIC SOY BROTH, PI 7164' REV vol. 06, November 2010, pages 1 - 3, XP055321116 Retrieved from the Internet: <URL:http://www.neogen.com/acumedia/pdf/Pro dInfo/7164_PI.pdf> [retrieved on 2015-01-19]**

**Description**

BACKGROUND

*Pseudomonas aeruginosa* is one of the leading causes of Gram-negative bacterial infections in the hospital setting (H. Fazeli, R. Akbari, S. Moghim, T. Narimani, M. R. Arabestani and A. R. Ghoddousi, J. Res. Med. Sci., 2012, 17, 332-337; V. H. Tam, K. T. Chang, K. Abdelraouf, C. G. Brioso, M. Ameka, L. A. McCaskey, J. S. Weston, J. P. Caeiro and K. W. Garey, Antimicrob. Agents Chemother., 2010, 54, 1160-1164). This opportunistic pathogen is frequently linked to patients with cystic fibrosis, (T. W. Lee, K. G. Brownlee, M. Denton, J. M. Littlewood and S. P. Conway, Pediatr. Pulmonol., 2004, 37, 104-110; J. B. Lyczak, C. L. Cannon and G. B. Pier, Clin. Microbiol. Rev., 2002, 15, 194-222; and F. Ratjen and G. Doring, Lancet, 2003, 361, 681-689) severe burn victims, and immunocompromised hosts such as patients with AIDS (J. B. Lyczak, C. L. Cannon and G. B. Pier, Microbes Infect., 2000, 2, 1051-1060). Although it is seldom responsible for infections in healthy individuals, several factors contribute to the success of *P. aeruginosa* in the compromised including its ability to thrive in the hospital environment and its increasing resistance to antibiotics (H. Fazeli, R. Akbari, S. Moghim, T. Narimani, M. R. Arabestani and A. R. Ghoddousi, J. Res. Med. Sci., 2012, 17, 332-337). *P. aeruginosa* infections pose serious concerns for this patient population, making it an important bacterium to study in the scientific and medical community.

Unique to *P. aeruginosa* is its production of pyocyanin, a redox-active quorum sensing molecule linked to biofilm formation and therefore a significant contributor in the bacterium's pathogenesis (E. Kipnis, T. Sawa and J. Wiener-Kronish, Med. Mal. Infect., 2006, 36, 78-91; G. W. Lau, D. J. Hassett, H. Ran and F. Kong, Trends Mol. Med., 2004, 10, 599-606; and L. E. Dietrich, A. Price-Whelan, A. Petersen, M. Whiteley and D. K. Newman, Mol. Microbiol., 2006, 61, 1308-1321). Because it is redox-active, pyocyanin can be detected electrochemically (T. A. Webster and E. D. Goluch, Lab Chip, 2012, 12, 5195-5201; V. B. Wang, S. L. Chua, B. Cao, T. Seviour, V. J. Nesatyy, E. Marsili, S. Kjelleberg, M. Givskov, T. Tolker-Nielsen, H. Song, J. S. Loo and L. Yang, PLoS One, 2013, 8, e63129; D. Sharp, P. Gladstone, R. B. Smith, S. Forsythe and J. Davis, Bioelectrochemistry, 2010, 77, 114-119; T. A. Webster, H. J. Sismaet and E. D. Goluch, Nano LIFE, 2013, 03, 1340011; and T. A. Webster, H. J. Sismaet, J. L. Conte, I. P. Chan and E. D. Goluch, Biosens. Bioelectron., 2014, 60, 265-270). Recently published literature has shown that the addition of amino acids up-regulates the biofilm formation of *P. aeruginosa* (S. P. Bernier, D. G. Ha, W. Khan, J. H. Merritt and G. A. O'Toole, Res. Microbiol., 2011, 162, 680-688). However, these studies do not address the link between pyocyanin production and *P. aeruginosa* growth. Research carried out in the 1950's to determine how amino acids influence pyocyanin production has not been revisited or applied to sensing strategies (E. Hellinger, J. Gen. Microbiol., 1951, 5, 633-639; N. Grossowicz, P. Hayat and Y. S. Halpern, J. Gen. Microbiol., 1957, 16, 576-583).

It is well-known that amino acids are the building blocks for protein synthesis and thus serve as key components for bacterial growth, such as peptidoglycan for cell wall formation (W. He, C. Li and C. D. Lu, J. Bacteriol., 2011, 193, 2107-2115). Thomas et al. found that amino acid concentrations in sputum were higher for those with more severe cases of cystic fibrosis, (S. R. Thomas, A. Ray, M. E. Hodson and T. L. Pitt, Thorax, 2000, 55, 795-797). Pierson et al. proposed a biosynthetic pathway for pyocyanin whose precursors include a branch-point synthesis of aromatic amino acids, lending credibility to using amino acids as regulatory molecules (L. S. Pierson, 3rd and E. A. Pierson, Appl. Microbiol. Biotechnol., 2010, 86, 1659-1670). As amino acid synthesis is a metabolically expensive process, it is possible that bacteria such as *P. aeruginosa* adapt their metabolic pathways given an abundance of free amino acids in the environment. Six amino acids (proline, histidine, arginine, leucine, tyrosine, and valine) are known to up-regulate biofilm formation in *P. aeruginosa* cultures (S. P. Bernier, D. G. Ha, W. Khan, J. H. Merritt and G. A. O'Toole, Res. Microbiol., 2011, 162, 680-688).

There remains a need to develop improved electrochemical techniques for early infection identification.

SUMMARY OF THE INVENTION

Devices for electrochemical measurement of a redox active substance secreted by a cell, methods of fabricating the devices, and methods of detecting a redox active substance using the devices are provided.

The embodiments and/or examples disclosed in the following description which are not covered by the appended claims are considered as not being part of the present invention.

As used herein, the term "nanoscale" refers to an object or a feature whose size is in the range from about 1 nm to about 999 nm, or to less than 1 μm. The term "microscale" refers to an object or feature whose size is in the range from about 1 μm to about 999 μm, or to less than 1 mm. A "nanofluidic channel" as used herein is a space generally in the form of a channel and having cross-sectional dimensions of nanoscale size. Other dimensions of a nanofluidic channel, such as its length can be of microscale size or greater, or can also be of nanoscale size. A "microfluidic channel" as used herein is a channel having all cross-sectional dimensions in the microscale range or greater.

One aspect of the invention is a nanofluidic electrode assembly for detecting a redox active substance secreted by a cell in a liquid sample, the nanofluidic electrode assembly including: a nanofluidic channel disposed in a substrate;

an inlet and an outlet port in fluid connection with the nanofluidic channel, the inlet and outlet ports configured to add and/or remove liquid from the liquid sample to or from the nanofluidic channel; a working electrode disposed in the nanofluidic channel, wherein the working electrode is suitable for applying potentials sufficient to alternately oxidize and reduce a redox active substance; a reference electrode disposed in the nanofluidic channel; and a matrix containing a chemical agent that stimulates secretion of the redox active substance by the cell, wherein the matrix is capable of releasing the chemical agent, and wherein the matrix is disposed in or on one or more of the nanofluidic channel, the inlet port, the outlet port, at least a portion of a surface of the working electrode, at least a portion of a surface of the reference electrode, and at least a portion of a surface of the substrate, the surface being adjacent to the inlet and/or outlet port; such that the redox active substance is detected as current flow through the working electrode.

The nanofluidic electrode assembly has a first and a second working electrode, the first working electrode being suitable for applying a potential sufficient to oxidize the redox active substance, and the second working electrode being suitable for applying a potential sufficient to reduce the redox active substance, and the matrix is disposed in or on one or more of the nanofluidic channel, the inlet port, the outlet port, at least a portion of a surface of the first working electrode, at least a portion of the surface of the second working electrode, at least a portion of a surface of the reference electrode, and at least a portion of a surface of the substrate, the surface being adjacent to the inlet and/or outlet port. In related embodiments, the first and the second working electrodes are separated by a distance of about 20 - 100 nm across the nanofluidic channel.

The matrix is made of a material that allows release of the chemical agent to the cell. In some embodiments, the matrix includes a polymer, a biocompatible polymer, a hydrogel, or a hydrophilic polymer. For example, the matrix may be made of gelatin, chitosan, alginate, fibrin, collagen, elastin, agar, agarose, hyaluronic acid, dextran, cellulose, poly(vinyl alcohol), polyacrylamide, poly(N-vinylpyrolidone), poly(hydroxyethyl methacrylate), poly(ethylene oxide), poly(ethylene glycol), poly(ethylene glycol) monomethyl ether, poly(acrylate), polymethacrylate, poly(methylacrylate), poly(methyl methacrylate) or poly(lactic acid).

The matrix is situated to allow release of the chemical agent to the cell. In some embodiments, the matrix is disposed in the nanofluidic channel adjacent to the connecting port(s). In some embodiments, the matrix is disposed in the microfluidic channel adjacent to the connecting port(s). In some embodiments, the matrix is attached to at least a portion of a surface of the working electrode. In some embodiments, the matrix is attached to at least a portion of a surface of the oxidizing working electrode. In some embodiments, the matrix is attached to at least a portion of a surface of the reducizing working electrode.

In some embodiments, the matrix contains one or more chemical agents that stimulate secretion of one or more redox active substances by one or more types of cells.

In various embodiments the working electrode(s) of the devices include a material selected from the group consisting of: gold, electrically conductive diamond, platinum, and glassy carbon.

In other related embodiments the substrate of the devices includes a glass having a refractive index greater than 1.5. For example, the refractive index is in the range of 1.6 - 1.9. For example, the glass has a thickness of about 100 nm.

Related embodiments include devices that further include integrated Complementary Metal-Oxide Semiconductor (CMOS) electronics and a wireless transmitter.

In some embodiments, the cell is a bacterium or fungus that secretes a siderophore, for example, one described by Hider and Kong (R. C. Hider and X. Kong, 2010, Nat. Prod. Rep. 27: 637-657). In some embodiments, the cell is a bacterium or fungus selected from a group consisting of *Ustilago sphaerogena, Streptomyces pilosus, Streptomyces coelicolor, Streptomyces coelicolor, Fusarium roseum, Burkholderia cepacia, Rhodotorula pilimanae, Escherichia coli, Bacillus subtilis, Bacillus anthracis, Vibrio cholera, Azotobacter vinelandii,*

*Pseudomonas aeruginosa,* and *Yersinia pestis.* In some embodiments, the cell is the bacterium *Pseudomonas aeruginosa.*

In some embodiments, the chemical agent is an amino acid, for example, alanine, arginine, asparagine, aspartic acid, cysteine, glutamine, glutamic acid, glycine, histidine, isoleucine, leucine, lysine, methionine, phenylalanine, proline, serine, threonine tryptophan tyrosine, or valine.

In some embodiments, the reference electrode includes Pd.

In some embodiments, the electrodes make up one or more walls of the nanofluidic channel. In some embodiments, the reference electrode forms at least a portion of a wall of the nanofluidic channel. In some embodiments, the working electrode forms at least a portion of a wall of the nanofluidic channel. In some embodiments, the first working electrode and/or second working electrode form at least a portion of a wall of the nanofluidic channel. In some embodiments for example, in the device having one working electrode, the reference electrode and the working electrode each form at least a portion of a wall of the nanofluidic channel and are separated by an insulating portion of the nanofluidic channel, the insulating portion extending from about 30 nm to about 50 $\mu$m along the length of the nanofluidic channel. In some embodiments for example, in the device having one working electrode, the reference electrode and the working electrode each form at least a portion of opposite facing walls of the nanofluidic channels, the opposite facing walls

separated by a distance of about 20 - 100 nm. In some embodiments for example, in the device having a first and a second working electrodes, the reference electrode and the first working electrode each form at least a portion of a wall of the nanofluidic channel and are separated by an insulating portion of the nanofluidic channel, the insulating portion extending from about 30 nm to about 50 μm along the length of the nanofluidic channel.
Some embodiments have a first and a second working electrode that form portions of opposite facing walls of the nanofluidic channel, the opposite facing walls separated by a distance of about 20 - 100 nm. In other related embodiments the reference electrode and the first and second working electrodes each form at least a portion of a wall of the nanofluidic channel, and the reference electrode is separated from both working electrodes by an insulating portion of the nanofluidic channel, the insulating portion extending from about 30 nm to about 50 μm along the length of the nanofluidic channel.

In some related embodiments, the working electrode(s) have a greater surface area than that of the reference electrode.

Embodiments described herein include a device having a working and a reference electrode, such that the working electrode has a surface area of at least 100 $nm^2$. In related embodiments, the area of the working electrode is less than 100,000, less than 50,000, less than 20,000, less than 10,000, or less than 500 $nm^2$.

In other embodiments, the working electrodes each have a surface area of at least 1 $\mu m^2$. In related embodiments, the area of each working electrode is less than 100, less than 50, less than 20, less than 10, or less than 5 $\mu m^2$.

In related embodiments the volume of the nanofluidic channel in which the electrodes are disposed is less than about 50 nanoliters (nL), less than about 10 nL, less than about 1 nL, less than about 100 picoliters (pL), less than about 50 pL less than about 10 pL, less than about 5 pL, or less than about 1 pL.

In some embodiments, the redox active substance is a siderophore secreted by a fungus or bacterium, for example, ferrichrome, Desferrioxamine B, Deferoxamine, Desferrioxamine E, fusarinine C, ornibactin, rhodotorulic acid, enterobactin, bacillibactin, vibriobactin, azotobactin, pyoverdine, yersiniabactin, or pyocyanin.

In some embodiments, the nanofluidic electrode assembly is configured for use as a medical device or a component of a medical device. In some embodiments, the nanofluidic electrode assembly is disposable. In some embodiments, the nanofluidic electrode assembly is suitable for implantation in a patient.

In some embodiments, at least a portion of the substrate of the nanofluidic electrode assembly is coated with a coating the promotes adhesion of the cell to the nanofluidic electrode assembly. In some embodiments the coating contains positively-charged ions, for example, poly-L-lysine.

Another aspect of the invention is a device including a nanofluidic electrode assembly and a counter electrode in fluid connection with the nanofluidic channel. For example, the counter electrode monitors the current flowing through the reference electrode and is useful to keep the current flow through the reference electrode to a low level, such as less than about InA. In some embodiments, the counter electrode is made of an inert material, for example, Pt or Hg. In some embodiments, the counter electrode forms at least a portion of a wall of the nanofluidic channel.

Another aspect of the invention is a device including a nanofluidic electrode assembly in electromagnetic connection with a processor that performs data analysis using the current flow through the working electrode.

Another aspect described herein is a device including a nanofluidic electrode assembly in electronic connection with a display that displays the current flow through the working electrodes or the concentration of the redox active substance.

Another aspect of the invention is a device including a plurality of nanofluidic electrode assemblies that allow for simultaneous detection of a plurality of redox active substances secreted by one or more types of cells. In some embodiments, the device has a plurality of nanofluidic electrode assemblies that have one working electrode. In some embodiments, the device has a plurality of nanofluidic electrode assemblies that have two working electrodes.

Another aspect of the invention is a device including a nanofluidic electrode assembly and a microfluidic channel in fluid connection with the nanofluidic channel of the nanofluidic electrode assembly via the inlet and outlet ports. In some embodiments, the matrix is disposed within the microfluidic channel adjacent to the inlet and/or outlet port. In some embodiments, the dimensions of the nanofluidic channel exclude passage of the cell from the microfluidic channel into the nanofluidic channel. In some embodiments, microfluidic channel has at least one entry port and at least one exit port in fluid connection with the microfluidic channel, the microfluidic entry port configured to add liquid to the microfluidic channel and the microfluidic exit port configured to remove liquid from the microfluidic channel. In some embodiments, the device includes a plurality of nanofluidic electrode assemblies in fluid connection with the microfluidic channel to allow for simultaneous detection of a plurality of redox active substances secreted by one or more types of cells.

One aspect of the invention is a device for detection of a redox active substance secreted by a cell in a liquid sample, the device including: a working electrode suitable for applying potentials sufficient to alternately oxidize and reduce the redox active substance; a matrix containing a chemical agent that stimulates secretion of the redox active substance by the cell, wherein the matrix is capable of releasing the chemical agent; and a substrate, to which the matrix is attached; wherein the cell and/or the redox active substance is detected as current flow through the working electrode.

One aspect described herein is a device for detection of a redox active substance secreted by a cell in a liquid sample, the device including: a working electrode suitable for applying potentials sufficient to alternately oxidize and

reduce a redox active substance; and a matrix containing a chemical agent that stimulates secretion of the substance by the cell, wherein the matrix is capable of releasing the chemical agent, the matrix being attached to at least a portion of a surface of the working electrode; wherein the cell and/or the redox active substance is detected as current flow through the working electrode.

In some embodiments the device includes a fluid compartment capable of containing the liquid sample in contact with the working electrode. In some embodiments, the fluid compartment is the substrate to which the matrix is attached.

Another aspect described herein is an electrochemical cell including a device for detection of a redox active substance secreted by a cell in a liquid sample and a reference electrode. In some embodiments, the electrochemical cell includes a counter electrode.

One aspect described herein is a method for fabricating a device for detection of a redox active substance secreted by a cell in a liquid sample, the method comprising depositing a matrix comprising a chemical agent that stimulates secretion of the redox active substance by the cell, wherein the matrix is capable of releasing the chemical agent, on at least a portion of a surface of a working electrode.

In some embodiments, the working electrode is disposed within a nanofluidic channel or a structure that is used to form a nanofluidic channel.

One aspect described herein is a method of fabricating a nanofluidic electrode assembly for detecting a redox active substance secreted by a cell in a liquid sample, the method including the steps of: depositing a first working electrode layer on a substrate; depositing a sacrificial layer on the first working electrode layer; optionally, depositing a second working electrode layer on a portion of the sacrificial layer; depositing a reference electrode layer on a portion of the sacrificial layer; depositing an insulating layer, wherein the insulating layer covers remaining exposed surfaces of the first working electrode layer, the sacrificial layer, the second working electrode layer if present, and the reference electrode layer; forming two or more holes through the insulating layer, the holes providing a fluid connection to the sacrificial layer and creating at least one inlet port and at least one outlet port; applying an etching agent through the holes, whereby the sacrificial layer is etched away to form a nanofluidic channel, wherein the first working electrode layer, the second working electrode layer if present, and the reference electrode layer each form at least a portion of a wall of the nanofluidic channel; depositing a matrix, wherein the matrix is capable of releasing a chemical agent that stimulates secretion of the redox active substance by the cell, into one or more of the nanofluidic channel, the inlet port, the outlet port, at least a portion of a surface of the first working electrode layer, at least a portion of a surface of the second working electrode layer if present, at least a portion of a surface of the reference electrode layer, and at least a portion of a surface of the substrate that is adjacent to the inlet port and/or outlet port.

In related embodiments the method further includes applying a resist material over one or more layers and performing lithography to create a patterned mask prior to applying a subsequent layer. Another related embodiment of the method includes bonding a wire to an electrode layer.

In some embodiments, the step of depositing a second working electrode layer is performed. In some embodiments, the step of depositing a second working electrode layer is not performed.

In some embodiments, the matrix is permeated with the chemical agent. In some embodiments, the matrix is permeated with the chemical agent prior to the step of depositing the matrix. In some embodiments, the matrix is permeated with the chemical agent subsequent to the step of depositing the matrix. In some embodiments the matrix is permeated with more than one chemical agent.

In some embodiments, the method further comprises the step of depositing on a surface of the insulating layer a coating that promotes adhesion of the cell to the nanofluidic electrode assembly. In some embodiments, the step of depositing the coating is performed subsequent the etching step and prior to the step of depositing the matrix. In some embodiments, the step of deposing the coating is performed subsequent the step of depositing the matrix.

Another aspect described herein is a method of fabricating a microfluidic-nanofluidic device including the steps of: separately, in any order, fabricating at least one nanofluidic electrode assembly and fabricating a microfluidic channel in a substrate; and combining the at least one nanofluidic electrode assembly with the at least one microfluidic channel so that the nanofluidic channel(s) are in fluid connection with the microfluidic channel via the inlet port(s) and outlet port(s). In various embodiments the microfluidic channels have forms selected from the group of: a channel, a reservoir, an open access port, and an open access port covered with a semipermeable membrane.

According to other related embodiments, in the method for fabricating a nanoscale device the thickness of the first electrode layer is about 20 nm. In other embodiments the thickness of the reference electrode layer is about 120 nm. Related embodiments include a method wherein the thickness of the sacrificial layer is about 20-200 nm. Further, in other related embodiments, the thickness of the insulating layer is about 500 nm. Embodiments include those in which the insulating layer includes silicon dioxide. Embodiments also include those in which the insulating layer includes a first layer of oxide, a layer of nitride, and a second layer of oxide. For example, the first and the second layer of oxide include $SiO_2$, and the layer of nitride includes $Si_3N_4$.

In various related embodiments, the method is such that the first electrode layer includes a material selected from the group consisting of: gold, electrically conductive diamond, platinum, and glassy carbon. In other related embodiments the reference electrode layer includes palladium. In some embodiments the electrode layers are deposited by a method selected from the group consisting of: electron beam deposition, physical vapor deposition, and chemical vapor deposition. In related embodiments the method further includes depositing a second electrode layer on the sacrificial layer, the second layer and the reference layer being essentially coplanar and non-adjacent. In various embodiments the substrate is a silicon wafer having a surface layer of $SiO_2$.

One aspect of the invention is a method of detecting a redox active substance secreted by a cell in a liquid sample, the method including: providing a nanofluidic electrode assembly described herein; exposing a liquid sample comprising a cell to the matrix of the device, wherein the chemical agent is released from the matrix and stimulates secretion of the redox active substance by the cell; charging a reference electrode to stabilize its potential; applying alternately a potential suitable for oxidizing the redox active substance and a potential suitable for reducing the redox active substance at the working electrode; and measuring current flow through the working electrode; wherein a current flow above a threshold value indicates a presence of the redox active substance, and a current flow below the threshold value indicates an absence of the redox active substance.

In some embodiments, the method includes: providing an nanofluidic electrode assembly having a first and a second working electrodes described herein; and applying a potential suitable for oxidizing the redox active substance at the first working electrode, and applying a potential suitable for reducing the redox active substance at the second working electrode.

One aspect of the invention is a method of simultaneously detecting a first and a second redox active substances secreted by one or more types of cells in a liquid sample, wherein each of the first and the second redox active substances has a distinct redox potential, the method including: providing a nanofluidic electrode assembly described herein; exposing a liquid sample comprising on or more cell types to the matrix of the device, wherein one or more chemicals agent are released from the matrix and stimulate(s) secretion of the two or more redox active substances by the one or more cell types; charging a reference electrode to stabilize its potential; applying alternately a first set of potentials suitable for oxidizing and reducing the first redox active substance at the working electrode and a second set of potentials suitable for oxidizing and reducing the second redox active substance at the working electrode; and measuring current flow through the working electrode; wherein a current flow above a threshold value during application of the first set of potentials indicates a presence of the first redox active substance, and a current flow below the threshold value indicates an absence of the first redox active substance; and wherein a current flow above a threshold value during application of the second set of potentials indicates a presence of the second redox active substance, and a current flow below the threshold value indicates an absence of the second redox active substance.

In some embodiments, the method of simultaneously detecting a first and a second redox active substances secreted by one or more types of cells in a liquid sample, wherein each of the first and the second redox active substances has a distinct redox potential, includes: applying alternately a first set of potentials suitable for oxidizing the first and the second redox active substances at the working and a second set of potentials suitable for reducing the first and the second redox active substances at the working electrode; wherein a current flow above a first threshold value during application of a first potential indicates a presence of the first redox active substance, and a current flow below the first threshold value indicates an absence of the first redox active substance; and wherein a current flow above a second threshold value during application of a second potential indicates a presence of the second redox active substance, and a current flow below the second threshold value indicates an absence of the second redox active substance.

In some embodiments, the method of simultaneously detecting a first and a second redox active substances secreted by one or more types of cells in a liquid sample, wherein each of the first and the second redox active substances has a distinct redox potential, includes:

providing an nanofluidic electrode assembly having a first and a second working electrodes described herein; applying a first set of oxidizing potentials suitable for oxidizing the first redox active substance and a second set of oxidizing potentials suitable for oxidizing the second redox active substance at the first working electrode, and applying first set of reducing potentials suitable for reducing the first redox active substance and a second set of reducing potentials suitable for reducing the second redox active substance at the second working electrode; and measuring current flow through the first and the second working electrodes; wherein a current flow above a first threshold value during application of the first sets of oxidizing and reducing potentials indicates a presence of the first redox active substance, and a current flow below the first threshold value indicates an absence of the first redox active substance; and wherein a current flow above a second threshold value during application of the second sets of oxidizing and reducing potentials indicates a presence of the second redox active substance, and a current flow below the second threshold value indicates an absence of the second redox active substance.

In some embodiments, the method of simultaneously detecting a first and a second redox active substances secreted by one or more types of cells in a liquid sample, wherein each of the first and the second redox active substances has a distinct redox potential, includes: applying a set of oxidizing potentials suitable for oxidizing the first and the second redox active substances at the first working electrode, and applying a set of reducing potentials suitable for reducing the first and the second redox active substances at the second working electrode; wherein a current flow above a first

threshold value during application of a first potential indicates a presence of the first redox active substance, and a current flow below the first threshold value indicates an absence of the first redox active substance; and wherein a current flow above a second threshold value during application of a second potential indicates a presence of the second redox active substance, and a current flow below the second threshold value indicates an absence of the second redox active substance.

In some embodiments, a third redox active substance is detected simultaneously, wherein a third set of potentials suitable for oxidizing and reducing the third redox active substance is applied at the working electrode, and wherein a current flow above a threshold value during application of the third set of potentials indicates a presence of the third redox active substance, and a current flow below the threshold value indicates an absence of the third active substance.

In some embodiments, the detection of the presence of the redox active substance indicates a presence of a cell that secretes the redox active substance, and detection of the absence of the redox active substance indicates an absence of the cell that secretes the redox active substance.

One aspect described herein is a method of detecting a redox active substance secreted by a cell in a liquid sample, the method including: providing a nanofluidic electrode assembly having a working electrode; contacting the liquid sample with the nanofluidic electrode assembly, wherein the liquid sample is fluidically connected with the inlet and outlet ports, and wherein the cell comes into a region proximal to the matrix, whereby the chemical agent from the matrix stimulates secretion of the redox active substance by the cell; charging the reference electrode to stabilize its potential; applying alternately a potential suitable for oxidizing the redox active substance and a potential suitable for reducing the redox active substance at the working electrode; recording current flow through the working electrode; and detecting the redox active substance by using a previously determined correlation between known concentrations of the redox active substance and the current flow through the working electrodes.

One aspect described herein is a method of detecting a redox active substance secreted by a cell in a liquid sample, the method including: providing a nanofluidic electrode assembly having a first and a second working electrodes described herein; exposing the liquid sample to the matrix, wherein the chemical agent is released from the matrix; contacting the liquid sample with the nanofluidic electrode assembly, wherein the liquid sample is fluidically connected with the inlet and outlet ports, and wherein the cell comes into a region proximal to the matrix, whereby the chemical agent from the matrix stimulates secretion of the redox active substance by the cell; charging the reference electrode to stabilize its potential; applying a potential suitable for oxidizing the redox active substance to the first working electrode and a potential suitable for reducing the redox active substance to the second working electrode, wherein the redox active substance has a halfway potential value between the potential of the first working electrode and the potential of the second working electrode; recording current flow through the first and second working electrodes; and detecting the redox active substance by using a previously determined correlation between known concentrations of the redox active substance and the current flow through the working electrodes.

In some embodiments, the liquid sample is a bodily fluid, for example, bronchial lavage, sputum, urine, saliva, spinal fluid, or blood. In some embodiments, the liquid sample is from a patient with cystic fibrosis.

In some embodiments, the method includes the step of determining the pH of the liquid sample using a pH electrode disposed within the nanofluidic channel, and applying a previously determined correlation between known amounts of the redox active substance in a medium of known pH and currents produced by the known amounts of the redox active substance.

In some embodiments, the current flow in the recording step is measured in response to one or more square wave potentials.

In some embodiments, the region proximal to the matrix is within a microfluidic channel.

## BRIEF DESCRIPTION OF THE DRAWINGS

FIG. 1 is schematic of an embodiment of a device of the invention having a working electrode, a matrix containing a chemical agent, and a substrate to which the matrix is attached.

FIG. 2 is a schematic of an embodiment of a device of the invention having a working electrode to which a matrix containing a chemical agent is attached.

FIG. 3 is a schematic of the fabrication scheme for fabrication of a nanofluidic electrode assembly having two working electrodes. Panels on the left represent a side view, and panels on the right represent an end view, showing (a) deposition of the first working electrode onto the insulating substrate, (b) deposition of the sacrificial layer, (c) deposition of the second working electrode, (d) deposition of the reference electrode, (e) deposition of the insulating layer, (f) boring access holes in the insulating layers, (g) removal of the sacrificial layers, and (h) deposition of the matrix.

FIG. 4 is a side-view representation of a nanofluidic electrode assembly having two working electrodes.

FIG. 5 is a top-view representation of a nanofluidic electrode assembly having one working electrode.

FIG. 6 is a top-view representation of a nanofluidic electrode assembly having two working electrodes.

FIG. 7 is a side-view representation of a nanofluidic electrode assembly having two working electrodes.

FIG. 8 is side-view representation of a device of the invention comprising a nanofluidic electrode assembly having two working electrodes and a microfluidic channel.

FIGS. 9A and B are square-wave voltammograms of *P. aeruginosa* cells grown for 10 hours in the presence of individual amino acids. Cells in FIGS. 9A were cultured in M63 minimal media, and cells in FIG. 9B were cultured in trypticase soy broth. Amino acids were used at the following concentrations (mM): proline, 27.2; histidine, 8; arginine, 4.8; leucine, 25.6; tyrosine, 3.2; and valine, 17.6.

FIGS. 10A and B are graphs showing the maximum current at about -0.25 V vs. Ag/AgCl due to production of pyocyanin by *P. aeruginosa* cells at various time points. Cells in FIG. 10A were cultured in M63 minimal media, and cells in FIG. 10B were cultured in trypticase soy broth. Amino acids were used at the following concentrations (mM): proline, 27.2; histidine, 8; arginine, 4.8; leucine, 25.6; tyrosine, 3.2; and valine, 17.6.

FIGS. 11A and B are square-wave voltammograms of *P. aeruginosa* cells grown in trypticase soy broth for eight hours in the presence of various concentrations of tyrosine and valine, respectively.

FIGS. 12A and B are graphs showing the maximum current at about -0.25 V vs. Ag/AgCl due to production of pyocyanin by *P. aeruginosa* cells at various time points. Cells in FIG. 12A and B were cultured in TSB supplemented in the presence of various concentrations of tyrosine and valine, respectively.

FIGS. 13A and B are square-wave voltammograms of *P. aeruginosa* cells grown in trypticase soy broth for eight hours. Cells in FIG. 13A were cultured in the presence or absence of 16 mM tyrosine, and cells in FIG. 13B were cultured in the presence or absence of 17.6 mM valine. Cells in the presence of supplemental amino acids were used at concentrations of 4, 20, 40, and 400 million cells per mL, and cells in the absence of supplemental amino acids were used at concentrations of 4 million cells per mL. Control samples that contained supplemental amino acids but no cells were used.

FIG. 14A and B are graphs showing the maximum current at about -0.25 V vs. Ag/AgCl due to production of pyocyanin by *P. aeruginosa* cells at various time points. Cells in FIG. 14A were cultured in the presence or absence of 16 mM tyrosine, and cells in FIG. 14B were cultured in the presence or absence of 17.6 mM valine. Cells in the presence of supplemental amino acids were used at concentrations of 4, 20, 40, and 400 million cells per mL, and cells in the absence of supplemental amino acids were used at concentrations of 4 million cells per mL. Control samples that contained supplemental amino acids but no cells were used.

FIG. 15 shows square-wave voltammograms of various bodily fluids supplemented with 5 uM pyocyanin. Bodily fluids tested were (A) bronchial lavage, (B) sputum, (C), urine (D), whole blood without additives, and (E) whole blood with sodium heparin. Blanks indicate samples of the bodily fluid that were not supplemented with pyocyanin.

FIG. 16A and B show square-wave voltammograms of supernatants from bacterial cultures after one day of growth. Cells in FIG. 16A were cultured in TSB, and cells in FIG. 16B were cultured in lysogeny broth. The following bacterial species were tested: *P. aeruginosa* that express CFP, *P. aeruginosa* that express YFP, *E. coli* that express GFP, E. *coli* that express YFP, *S. aureus, S. epidermis,* and *B. cereus.*

## DETAILED DESCRIPTION OF THE INVENTION

The present invention provides devices and methods for highly sensitive electrochemical detection of redox active substances secreted by a cell. In some embodiments, the substances can be detected in picoliter volumes of liquid sample. The devices use an electrode and a matrix capable of releasing a chemical agent that can stimulate secretion of the redox active substance by the cell. In some embodiments, the device is a nanofluidic electrode assembly having a reference electrode and one or more working electrodes integrated within a nanofluidic channel. The invention further provides sensor devices that incorporate the nanofluidic electrode assembly, combinations of such sensor devices, medical devices incorporating the sensor devices, as well as methods for using the devices.

Methods described herein include methods for the fabrication of an electrode coupled with a matrix and methods for fabrication of a nanofluidic electrode assembly that includes a matrix. Methods of the invention also include methods of using the devices, including an electrode coupled with a matrix, an electrochemical cell that includes an electrode and a matrix, and a nanofluidic electrode assembly, to detect redox active substances. By utilizing suitable voltammetry protocols, a redox active substance can be selectively detected in a complex solution without the use of any electrode surface modification.

Microfabricated nanofluidic electrode assemblies having an integrated palladium reference and two closely spaced working electrodes (an oxidizing working electrode and a reducing working electrode) inside the device nanochannel are also described herein. One such device has been used to monitor changes in pyocyanin concentration voltammetrically in real time. A linear response in faradaic current ($R^2 = 0.96$) was observed over a biomedically relevant range of pyocyanin concentrations (0-100 $\mu$M) while continuously measuring the current for 2 hours. Measurement of

the current that results from the repeated oxidation and reduction of pyocyanin at two closely spaced electrodes inside the device nanochannel yielded a 1.07 μM limit of detection without electrical isolation of the electrochemical cell. Since a reference electrode is integrated inside the nanofluidic channel of these sensors, they can potentially be employed to detect pyocyanin and other redox-active molecules in wide range of medical and environmental settings where space is limited, and without the risk of a biofilm forming on the reference or other electrodes. Nanofluidic electrode assemblies can also be used with an external Ag/AgCl reference electrode, e.g., to determine the concentration of a redox active substance in a solution, such as a patient sample or a culture medium. This type of analysis is completed in less than two minutes and also has a sub micromolar detection limit.

Voltammetric detection provides a simple means of measuring the concentration of an electro-active species in solution. This is done by applying to an electrode a potential that oxidizes or reduces the molecule of interest. As the molecule of interest interacts with the electrode surface, one or more electrons are transferred between the two, resulting in the flow of a measurable current. Numerous compounds, including histamine, glutathione and dopamine, have been successfully detected using this approach (Lacher et al., 2001, Electrophoresis 22, 2526-2536; Dam et al., 2007, Analyst 132, 365-370; Pihel et al., 1995, Anal Chem 67, 4514-4521). However, often, the molecule of interest is in a complex medium that contains multiple electro-active species that react at the same potential, thus requiring that electrochemical detection be coupled with a separation technique, such as liquid chromatography (LC) or capillary electrophoresis (CE). While improving selectivity and sensitivity, these approaches also require additional processing time and reagents, and increase the device footprint. In certain situations, this can be avoided. If the molecule of interest is capable of both accepting and donating an electron at least once, a technique known as redox cycling can be employed to amplify the measured current over unstable molecules.

Redox cycling typically uses two working electrodes, with the potential of one electrode set to oxidize the target molecule, while the second electrode is set to a reducing potential. When the target molecule interacts with the oxidizing electrode it donates an electron, and when it interacts with the reducing electrode it gains the electron back and is able to repeat the process. This cycling process significantly increases the signal compared to a single working electrode system. Straver et al. (2012) recently demonstrated this effect by constructing a microfluidic electrode assembly where the spacing between the top and bottom electrodes was on the order of 1 micron (Straver, et al., Lab Chip 12, 1548-1553).

The theoretical limiting current for a given concentration of an electrochemical molecule undergoing redox cycling in a confined channel is determined by

$$i = \frac{nFSDC}{z} \qquad (1)$$

Where *i* is the limiting current, *n* is the number of electrons being transferred, F is Faraday's constant, S is the surface area that overlaps between the two electrodes, D is the diffusion constant of the molecule being studied, C is the concentration of the redox species, and z is the distance between the two electrodes (Goluch et al. 2009, Anal Bioanal Chem 394, 447-456). Equation 1 shows that for a given molecule, the net current measured at an electrode can be increased by either increasing the area that overlaps between electrodes or decreasing the spacing between electrodes. Decreasing the vertical spacing between the two electrodes (i.e. the two electrodes are placed on top of one another), increases the net current response of these devices (Goluch et al. 2009, Anal Bioanal Chem 394, 447-456). The benefit of decreasing the spacing between electrodes over increasing the area of overlap is that it allows for smaller device footprints.

Electrochemical detection systems utilizing redox cycling in nanofluidic channels have been shown to have increased sensitivity over their large scale counterparts, and have even been shown to detect single molecules (Zevenbergen et al., 2007, Nano Lett 7, 384-388; Zevenbergen et al., 2011, Nano Lett 11, 2881-2886). The ease of use of this method lends itself to the detection of biologically relevant molecules (Goluch et al., 2009, Anal Bioanal Chem 394, 447-456; Wolfrum, et al., 2008 Anal Chem 80, 972-977; Webster and Goluch, 2012 Lab Chip 12, 5195-5201). However, none of these earlier studies has utilized an integrated reference electrode. In contrast, the present invention provides a microfabricated electrochemical sensor with an integrated reference electrode. The entire footprint of the sensor, including the entire electrode assembly, can be, for example, as small as approximately 15 μm by 250 μm.

An electrochemical cell used for potentiometric analysis typically consists of three electrodes: a working electrode, a reference electrode, and a counter electrode. Measurements are made at the surface of the working electrode and the resulting potential is compared against the half-reaction taking place at the reference electrode. An ideal reference electrode is constructed from a material that has a high current exchange density, is nonpolarizable, and is not destroyed by the reaction taking place. The counter electrode is employed to ensure that the current passing through the reference electrode is never large enough to damage the electrode. When small currents are measured at the working electrode, such as less than 1 nA, the reference electrode can also serve as a counter electrode, allowing a two electrode setup. The current flowing through the working electrode, which can be measured using an voltammetry circuit that is part of

the device or a separate component, can be used as a measure of the concentration or amount of the selected analyte.

By decreasing the size of the electrode assembly, many individually addressable electrode assemblies can be packaged onto a single detection platform. By constructing electrode assemblies spaced less than 100 nm apart, it has been shown that the sensitivity of electrochemical devices can be increased several-fold as a result of the short molecular transport times between electrodes in these confined spaces (Wolfrum et al., 2008, Anal Chem, 80, 972-977; Zevenbergen et al., 2007, Nano Lett, 7, 384-388; Zevenbergen et al., 2011, Nano Lett, 11, 2881-2886). However, miniaturization, stability, and reliability of the reference electrode have only recently been addressed (Goluch, Edgar, D., and Webster, T., WO2014015333).

An exemplary use of the nanofluidic electrode assembly described herein is detection of a microbe, such as a human pathogen, by detection of a redox active compound released from the microbe. Ventilator-associated pneumonia is a prevalent affliction that plagues many immune-compromised patients in hospitals, and high mortality rates have been observed. A significant contributor to ventilator-associated pneumonia as well as wound and burn victim infections is the opportunistic pathogen *Pseudomonas aeruginosa* (PA). One of the many toxins produced and secreted by *Pseudomonas aeruginosa* is pyocyanin:

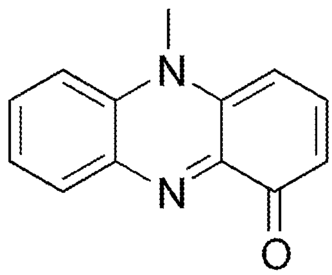

Pyocyanin has the ability to oxidize and reduce other molecules and has been shown to induce neutrophil apoptosis (programmed cell death), decreased glutathione levels in epithelial cells, cause oxidative stress to cells, and inhibit the beating of lung cilia, all of which allow bacterial infections to thrive and increase the prevalence of chronic infections.

Currently, the most common method for identification of microbial pathogens is culturing colonies, which takes 24 hours or more. Polymerase chain reaction (PCR) based assays, though quicker, still takes over an hour and require expensive reagents. In the case of *Pseudomonas aeruginosa* pulmonary infections, the sputum of patients can contain up to 130 μM concentrations of pyocyanin, and concentrations in the millimolar range have been reported in ear secretions obtained from patients with *Pseudomonas aeruginosa* ear infections. These concentrations make it possible to use pyocyanin as a diagnostic marker in electrochemical sensing techniques. Since the pyocyanin molecule is redox active, it can be detected electrochemically. Carbon electrodes were used by Sharp et al (2010) to selectively detect pyocyanin in bulk PA cultures; however the large size of the electrodes does not lend itself to microfluidic systems (Sharp et al., 2010, Bioelectrochem, 77, 114-119).

The devices and methods offer several advantages over existing methods for detecting microbial pathogens. First, embodiments of the nanofluidic electrode assembly described herein can be used for monitoring the production of pyocyanin by several strains of PA using only a few hundred microliters of sample. In addition, the use of a matrix containing a chemical agent that stimulates production of a redox active substance increases the sensitivity of detection. The chemical agent promotes secretion of the redox active substance by the cell, allowing for more rapid detection. Providing the chemical agent alone, however, could result in increased levels of the redox active substance and/or microbe, which could be deleterious if used in a medical device in continuous contact with a patient's bodily fluids. These potential side effects are alleviated by incorporating the chemical agent into a matrix, which allows the chemical agent to be released at high local concentrations near the electrode without causing systemic changes in levels of the redox active substance and/or microbe. Sensor devices employing such nanoelectrode assemblies can be incorporated into medical devices such as respirators, or disposable components thereof, such as tubes or masks. The sensors can be connected to control or monitoring electronic devices either by direct electrical connections or by wireless transmitter, and can provide important patient monitoring information in real time, or at selected times or time intervals.

The invention includes a device for detecting a redox active substance secreted by a cell in a liquid sample. As shown in FIG. 1, the device includes an electrode (**110**) and a matrix (**390**) that contains a chemical agent (**130**). In the embodiment shown in FIG. 1, the matrix is attached to a substrate (**140**), and the electrode is attached to an electrical lead (**150**). The electrode, matrix, and substrate are housed in a housing (**160**). The device may also include a fluid compartment (not shown) that can contain the liquid sample. In an alternate embodiment, shown in FIG. 2, the matrix is attached to the electrode.

The electrode can be part of a working electrode in an electrochemical cell used for voltammetry. For example, the electrode can be a working electrode in a two-electrode system that includes a second electrode that maintains a

constant potential and passes current to counter the redox events at the working electrode. Alternatively, the electrode can be used as a working electrode in a three-electrode system that includes a reference electrode that maintains a constant potential and a counter electrode that passes current. In another embodiment, the electrode can used as one working electrode in a three-electrode system that includes a second working electrode and a reference electrode.

A redox active substance is a molecule that can cycle between reduced and oxidized states. The redox active substance may be a siderophore secreted by a fungus or bacterium, for example, a siderophore described by Hider and Kong (R. C. Hider and X. Kong, 2010, Nat. Prod. Rep. 27: 637-657). The siderophore may be ferrichrome, Desferrioxamine B, Deferoxamine, Desferrioxamine E, fusarinine C, ornibactin, rhodotorulic acid, enterobactin, bacillibactin, vibriobactin, azotobactin, pyoverdine, yersiniabactin, or pyocyanin.

The matrix is a porous polymeric composition through which an aqueous solution can flow and from which a chemical agent can be released. The matrix may include a polymer, a biocompatible polymer, a hydrogel, or a hydrophilic polymer. For example, the matrix may be made of gelatin, chitosan, alginate, fibrin, collagen, elastin, agar, agarose, hyaluronic acid, dextran, cellulose, poly(vinyl alcohol), polyacrylamide, poly(N-vinylpyrolidone), poly(hydroxyethyl methacrylate), poly(ethylene oxide), poly(ethylene glycol), poly(ethylene glycol) monomethyl ether, poly(acrylate), polymethacrylate, poly(methylacrylate), poly(methyl methacrylate) or poly(lactic acid). The matrix may contain one or more chemical agents.

The chemical agent is an organic molecule or molecular complex that stimulates a cell to release a redox active substance. The chemical agent may be an organic molecule or molecular complex that stimulates a fungus or bacterium to secrete a redox active substance, for example, a siderophore. The chemical agent may be an amino acid, for example, alanine, arginine, asparagine, aspartic acid, cysteine, glutamine, glutamic acid, glycine, histidine, isoleucine, leucine, lysine, methionine, phenylalanine, proline, serine, threonine tryptophan tyrosine, or valine.

The application also encompasses an electrochemical cell that includes a working electrode, reference electrode, and matrix containing a chemical agent. The reference electrode may contain palladium (Pd), for example, PdH. The electrochemical cell may also contain a counter electrode. The counter electrode may be made of an inert material, for example, platinum (Pt) or mercury (Hg). The electrochemical cell may also contain a fluid compartment that can contain the liquid sample.

The application includes an electrochemical cell that can promote redox reactions. As shown in FIG. 3, a redox active substance (**400**) comes in contact with a first electrode (**510**), which applies a potential sufficient to oxidize the redox active substance. The redox active substance then comes in contact with a second electrode (**520**), which supplies a potential sufficient to reduce the redox active substance. As these steps are repeated, the redox active substance cycles back and forth between oxidized and reduced states. The first and second electrodes may provide oxidizing and reducing potentials, respectively, or the roles may be reversed.

The first electrode may be the working electrode in a two-electrode system, and the second electrode may be the electrode that maintains a constant potential and a passes current. In this embodiment, the potential applied by the working electrode may be alternated between an oxidizing and a reducing potential. In another embodiment, the first electrode is the working electrode and the second electrode is the counter electrode in a three-electrode system that also includes a reference electrode. In another embodiment, the first electrode is the first working electrode and the second electrode is the second working electrode in a three-electrode system that also includes a reference electrode.

The invention also includes a device for detecting a redox active substance secreted by a cell in a liquid sample. FIG. 4 shows an embodiment of such a device and its use for detecting a redox active substance secreted by a cell in a liquid sample. As a liquid sample containing a cell (**1790**) flows through a microfluidic channel (**1770**), the cell comes into a region proximal to the matrix (**390**). The cell may come into direct contact with the matrix, or it may pass near enough to the matrix, e.g., 10 $\mu$m, that the cell is exposed to a high local concentration of the chemical agent (not shown) diffusing from the matrix. Exposure to the chemical agent causes the cell to secrete the redox active substance (**400**), which undergoes a reduction-oxidation cycle at the first (**510**) and second (**520**) electrodes. The increase in concentration of the redox active substance at the electrodes is detected as a change in current.

The liquid sample may be contacted directly with the matrix. Alternatively, the liquid sample may be processed prior to contacting the matrix. For example, the liquid sample may altered chemically (e.g., by dilution, chemical reaction), physically (e.g., by centrifugation, radiation, etc.), biologically (e.g., by treatment with a virus, microbe, antibody, etc.), biochemically (e.g., by treatment with an enzyme), or by any other means.

The liquid sample may be any sample containing a cell that secretes a redox active substance. For example, the liquid sample may be a bodily fluid from a subject that may be infected with such a cell. For example, the liquid sample may be bronchial lavage, sputum, urine, saliva, spinal fluid, or blood. The subject may be a patient with cystic fibrosis.

The cell may be any cell that secretes a redox active substance. For example, the cell may be bacterium or fungus that secretes a siderophore, for example, one described by Hider and Kong (R. C. Hider and X. Kong, 2010, Nat. Prod. Rep. 27: 637-657). The cell may be a bacterium or fungus selected from one of the following: *Ustilago sphaerogena, Streptomyces pilosus, Streptomyces coelicolor, Streptomyces coelicolor, Fusarium roseum, Burkholderia cepacia, Rho-*

*dotorula pilimanae, Escherichia coli, Bacillus subtilis, Bacillus anthracis, Vibrio cholera, Azotobacter vinelandii, Pseudomonas aeruginosa,* and *Yersinia pestis.*

The device may include a coating that promotes adhesion of the cell in contact with or in a region proximal to the matrix. In one embodiment, shown in FIG. 4, the coating (**1760**) lies within the microfluidic channel, although other arrangements are possible. By trapping the cell or delaying its progress through the channel, the coating increases the exposure of the cell to the chemical agent, resulting in increased secretion of the redox active substance. Consequently, the coating improves the sensitivity of the device. The coating may be any material that promotes adhesion of the cell. For example, the coating may be a positively-charged polymer, e.g., poly-L-lysine.

The invention includes a nanofluidic electrode assembly for detecting a redox active substance secreted by a cell in a liquid sample. The nanofluidic electrode assembly has a nanofluidic channel within a substrate. At one end of the nanofluidic channel is at least one inlet port that allows the liquid sample to enter the nanofluidic channel. At the other end of the nanofluidic channel is at least one outlet port that allows the liquid sample to exit the nanofluidic channel. Contained within the nanofluidic channel are a first and second electrode. The electrodes may make up a wall or part of a wall of the nanofluidic channel. The first and second electrode are positioned on opposite walls of the nanofluidic channel so that the distance across the channel and between the electrodes is in the range from about 20 nm to about 100 nm, or from about 20 nm to about 40 nm, or from about 40 nm to about 60 nm, or from about 60 nm to about 80 nm, or from about 80 nm to about 100 nm, or from abut 100 nm to about 150 nm. The surface area of the working electrodes can be selected according to the design priorities of the device, although having a larger surface area improves the signal and sensitivity of the device. For example, in different embodiments, the surface area of each working electrode can be about 100, 200, 300, 400, 500, 800, 1000, 2000, 3000, 5000, 10000, 50000, 100000, 200000, or 500000 $nm^2$; or 1, 2, 5, or 10 $\mu m^2$ or greater. The volume of the nanofluidic channel in which the electrodes are positioned may be less than about 50 nanoliters (nL), less than about 10 nL, less than about 1 nL, less than about 100 picoliters (pL), less than about 50 pL less than about 10 pL, less than about 5 pL, or less than about 1 pL. The nanofluidic electrode assembly also includes a matrix capable of releasing a chemical agent. FIG. 5 shows an embodiment of a nanofluidic electrode assembly having a first (**510**) and second (**520**) electrode within the nanofluidic channel. The matrix (**390**) is permeable to the redox active substance (**400**), which allows the redox active substance to diffuse into the nanofluidic channel to contact the electrodes.

The matrix may be situated anywhere on the nanoscale electrode assembly that allows a cell to come into a region proximal to the matrix. For example, the matrix may be attached to one or more of the following sites: the nanofluidic channel; the inlet port, the outlet port; a surface of the reference electrode, or a portion thereof; a surface of the working electrode(s), or a portion thereof; and a surface of the substrate, the surface being adjacent to the inlet port(s) or outlet port, or a portion of such a surface.

In one embodiment, the nanofluidic electrode assembly has a working electrode and a reference electrode, as shown in FIG. 6. The working electrode (**310**) and reference electrode (**350**) each contact the nanofluidic channel (not visible from top view). The access holes (**370**) to the nanofluidic channel serve as inlet and outlet ports when the liquid sample is applied to the nanofluidic electrode assembly. The matrix (**390**) may be positioned in and adjacent to the inlet and outlet ports, although other arrangements are possible. In some embodiments, the working and reference electrodes form portions of opposite facing walls of the nanofluidic channel, the opposite facing walls separated by a distance of about 20 - 100 nm across the channel. In some embodiments the reference electrode and the working electrode each form at least a portion of a wall of the nanofluidic channel and are separated by an insulating portion of the nanofluidic channel, the insulating portion extending from about 30 nm to about 50 $\mu m$ along the length of the nanofluidic channel. The reference and working electrodes also can be on opposite walls of the nanofluidic channel, but the electrodes should not touch each other. In some embodiments, the working electrode(s) have a greater surface area than that of the reference electrode. The working electrode and reference electrode may each have a surface area of at least 100 $nm^2$ or of less than 100,000, less than 50,000, less than 20,000, less than 10,000, or less than 500 $nm^2$. In one embodiment, this nanofluidic electrode assembly can operate as a two-electrode electrochemical cell. In another embodiment the nanofluidic electrode assembly includes a counter electrode so that the system operates as a three-electrode electrochemical cell. The counter electrode may be included in the nanofluidic channel, or it may be external.

In another embodiment, the nanofluidic electrode assembly has two working electrodes and a reference electrode, as shown in FIG. 7. The first working electrode (**310**), second (**340**) working electrode, and reference electrode (**350**) each contact the nanofluidic channel (**380**). The matrix (**390**) may be positioned in and adjacent to the access holes (**370**), which serve as inlet and outlet ports, although other arrangements are possible. The first and second working electrode form portions of opposite facing walls of the nanofluidic channel, the opposite facing walls separated by a distance of about 20 - 100 nm across the channel. In other related embodiments the reference electrode and the working electrodes each form at least a portion of a wall of the nanofluidic channel, and the reference electrode is separated from both working electrodes by an insulating portion of the nanofluidic channel, the insulating portion extending from about 30 nm to about 50 $\mu m$ along the length of the nanofluidic channel. In some embodiments, the working electrodes each have a surface area of at least 1 $\mu m^2$ or less than 100, less than 50, less than 20, less than 10, or less than 5 $\mu m^2$.

The nanofluidic electrode assembly may also include a coating that promotes adhesion of the cell in contact with or in a region proximal to the matrix. In one embodiment, shown in FIG. 4, the coating (**1760**) is attached to the insulating layer (**360**), although other arrangements are possible.

The nanofluidic electrode assembly may be in electromagnetic communication with a processor that can analyze data and perform calculations based on the current flow through the working electrode. The processor may be wired to the nanofluidic electrode assembly to receive current measurements from the working electrode and other information about the liquid sample. Alternatively, the processor may be connected to receive current measurements from the working electrode and other information about the liquid sample via a wireless mechanism.

The nanofluidic electrode assembly may be in electromagnetic communication with a display that can display the current flow through the working electrode. The display may be wired to the nanofluidic electrode assembly to receive current measurements from the working electrode. Alternatively, the display may receive current measurements from the working electrode via a wireless mechanism.

The nanofluidic electrode assembly may be attached to a microfluidic channel through which the liquid sample can pass. As shown in FIG. 4, the microfluidic channel (**1770**) is fluidically connected with the inlet and outlet ports of the nanofluidic channel. As the liquid sample flows through the microfluidic channel, contents of the sample are able to enter the nanofluidic channel via the inlet port and exit the nanofluidic channel via the outlet port. Thus, any redox active substance in the liquid sample is able to enter the nanofluidic channel and come in contact with the working electrode(s) and reference electrode. The microfluidic channel may have one or more entry ports (**1800**) and one or more exit ports (**1810**) that allow the liquid sample to enter and exit, respectively, the microfluidic channel. The dimensions of the microfluidic and nanofluidic channels may be such that the cell that secretes the redox active substance is able to pass through the microfluidic channel but unable to pass through the nanofluidic channel. For example, the nanofluidic channel may have a height and/or width of less than 200 nm, or less than 500 nm, or less than 700 nm, or less than 800 nm, or less than 900 nm, or less than 1000 nm.

The invention includes devices for simultaneous detection of a plurality of redox active substances secreted by one or more types of cells in a liquid sample. In one embodiment, such a device includes multiple nanofluidic electrode assemblies for processing the liquid sample. In one embodiment, such a device includes multiple nanofluidic electrode assemblies in fluid connection with a microfluidic channel.

Described herein is a method of fabricating a device for detecting a redox active substance secreted by a cell in a liquid sample. In one embodiment, the method includes the steps of depositing on a surface an electrode a matrix that can release a chemical agent. In another embodiment, the method includes depositing a matrix on a substrate. In one embodiment, the method involves first attaching the matrix to the working electrode or the substrate and then permeating the matrix with the chemical agent. In another embodiment, the matrix is first permeated with the chemical agent and then attached to the working electrode or the substrate.

The invention includes a method of fabricating a nanofluidic electrode assembly for detecting a redox active substance secreted by a cell in a liquid sample. As shown in FIG. 8, the first step entails depositing a first working electrode layer (**310**) on a substrate (**320**),
The working electrode layer may be gold, electrically conductive diamond, platinum, or glassy carbon. The substrate layer may be a silicon wafer having a surface layer of $SiO_2$. The working electrode layer and subsequent electrode layers may be deposited by electron beam deposition, physical vapor deposition, or chemical vapor deposition.

In the next step, a sacrificial layer (**330**) is deposited on the first electrode layer. The sacrificial layer may be made of chromium (Cr).

In the next step, a second working electrode layer (**340**) may be deposited on the sacrificial layer. In one embodiment of the method, this step is performed. In another embodiment of the method, this step is not performed. The second working electrode layer, if present, may extend for less than the full length of the sacrificial layer.

In the next step, the reference electrode layer (**350**) is deposited on the sacrificial layer. The reference electrode layer may extend for less than the full length of the sacrificial layer. If a second working electrode layer is present, the reference electrode layer is essentially coplanar and non-adjacent to the second working electrode layer. The gap between the reference electrode and second working electrode layers may be from about 30 nm to about 50 $\mu$m.

In the next step, an insulating layer (**360**) is deposited on the exposed surfaces of the substrate, first working electrode layer, sacrificial layer, second working electrode layer if present, and reference electrode layer. The insulating layer may be made of the same material used for the substrate layer, or they may be made of different materials. The insulating layer may be made of $SiO_2$.

In the next step, access holes (**370**) are bored into the insulating layer down to the sacrificial layer. During fabrication of the nanofluidic electrode assembly, the access holes allow fluid access to the sacrificial layer. In the completed nanofluidic electrode assembly, the access holes serve as inlet and outlet ports for fluid flow through the nanofluidic channel.

In the next step, the sacrificial layer is removed to create a nanofluidic channel (**380**). This can be accomplished, for example, by applying an etching agent through the access holes. In the next step, a matrix (**390**) is deposited onto

the nanofluidic electrode assembly. In the embodiment of the method shown, the matrix is added into the access holes and adjacent regions of the insulating substrate. However, the matrix can be added to one or more of the following sites: the nanofluidic channel; the inlet port, the outlet port; a surface of the reference electrode, or a portion thereof; a surface of the working electrode(s), or a portion thereof; and a surface of the substrate, the surface being adjacent to the inlet port(s) or outlet port, or a portion of such a surface. The matrix can be added by any means that will allow it to form and attach to the desired region of the nanofluidic electrode assembly. For example, it can be added as an aqueous solution, non-aqueous solution, or gel. It can be a polymer formed by changes in pH, changes in temperature, chemical reactions, electromagnetic radiation, or any other means.

The invention includes methods of using a device or electrochemical cell described above to detect a redox active substance secreted by a cell in a liquid sample. The first step entails providing a device of the invention, for example, a nanofluidic electrode assembly having either one or two working electrodes. If the device does not include a reference electrode, an external reference electrode may be provided.

In the next step, the liquid sample is contacted with the matrix, causing the cell in the liquid sample to be exposed to the chemical agent in the matrix. The liquid sample may be contacted directly with the matrix. Alternatively, the liquid sample may be processed prior to contacting the matrix. For example, the liquid sample may altered chemically (e.g., by dilution, chemical reaction), physically (e.g., by centrifugation, radiation, etc.), biologically (e.g., by treatment with a virus, microbe, antibody, etc.), biochemically (e.g., by treatment with an enzyme), or by any other means. Exposure of the cell in the liquid sample causes the cell to secrete a redox active substance, which diffuses to the electrode.

In the next step, the reference electrode is charged to stabilize its potential. In some embodiments, the potential is determined by the chemical half-reaction that occurs at the reference electrode.

In the next step, voltammetric potentials capable of oxidizing and reducing the redox active substance are alternately applied through the working electrode. Any type of a voltammetric changes in potential may be used, for example, linear sweep voltammetry, staircase voltammetry, square-wave voltammetry, cyclic voltammetry, or others. Potentials may be applied as a set of more than potentials, for example, potentials within a defined voltage range or alternating potentials defined by a mathematic relationship. A set may contain a single potential or multiple potentials.

In the next step, current flow through the working electrode is measured. This step allows the presence and/or concentration of the redox active substance in the liquid sample to be determined. Using known concentrations of the redox active substance, the relationship between voltammetric current and concentration of the redox active substance can be established under defined conditions of temperature, pH, presence of other electrolytes, and the like. By applying this relationship to the current measured the working electrode, the presence and/or concentration of the redox active substance in the liquid sample can be determined.

In some embodiments, the method includes determining the presence and/or concentration of multiple redox active substances. Redox active substances that have different redox potentials produce peaks of current at different applied potentials and therefore can be distinguished on a single voltammogram. As described above, the relationship between voltammetric current and concentration can be independently determined for each redox active substance under defined conditions. By applying these relationships to the current measured at the working electrode, the presence and/or concentration of multiples redox active substances in a liquid sample can be determined. This can be accomplished by applying a separate set of potentials for each redox active agent. Alternatively, it can be accomplished by applying one set of potentials that elicits separate peaks in current for each redox active agent. In an embodiment of the method that employs a device with one working electrode, the oxidizing and reducing potentials can be applied alternately from the working electrode. In an embodiment of the method that employs a device with two working electrodes, the oxidizing and reducing potentials can be applied from separate working electrodes.

In some embodiments, the method includes the step of determining the presence and/or concentration of cells that secrete the redox active substance in the liquid sample. By using a known number and/or concentration of cells in a liquid sample and measuring the amount of redox active substance produced under defined conditions, the relationship between cell number and amount of redox active substance can be determined. This relationship, taken together with the relationship between the voltammetric current and amount of redox active substance determined as described above, can be used to determine the relationship between voltammetric current and cell number.

The invention includes methods of using a nanofluidic electrode assembly to detect a redox active substance secreted by a cell in a liquid sample. In the first step, the liquid sample is contacted with the nanofluidic electrode assembly so that the sample forms a fluid connection with the inlet and outlet ports, as shown in FIG. 4. The liquid sample may be contacted directly with the nanofluidic electrode assembly. Alternatively, the liquid sample may be processed prior to contacting the matrix. For example, the liquid sample may altered chemically (e.g., by dilution, chemical reaction), physically (e.g., by centrifugation, radiation, etc.), biologically (e.g., by treatment with a virus, microbe, antibody, etc.), biochemically (e.g., by treatment with an enzyme), or by any other means. As the liquid sample contacts the nanofluidic electrode assembly, a cell (**1790**) in the liquid sample comes into a region proximal to the matrix (**390**). The cell may come into direct contact with the matrix, or it may pass near enough to the matrix, e.g., 10 $\mu$m, that the cell is exposed to a high local concentration of the chemical agent (not shown) diffusing from the matrix. Exposure to the chemical agent

causes the cell to secrete the redox active substance (**400**).

In the next step, the reference electrode (not shown) is charged to stabilize its potential, as described above.

In the next step, voltammetric potentials capable of oxidizing and reducing the redox active substance are applied to the working electrode(s). For embodiments of the method involving a nanofluidic electrode assembly with one working electrode, voltammetric potentials capable of oxidizing and reducing the redox active substance are alternately applied through the working electrode. For embodiments of the method involving a nanofluidic electrode assembly with two working electrodes, voltammetric potentials capable of oxidizing the redox active substance are applied through one working electrode, and voltammetric potentials capable of reducing the redox active substance are applied through the second working electrode. As shown in FIG. 4, the first (**510**) and second (**520**) electrodes can serve as the first and second working electrodes in the nanofluidic electrode assembly having two working electrodes. The first working electrode may provide the oxidizing potential and the second working electrode may provide the reducing potential, or vice versa. Any type of a voltammetric changes in potential may be used, for example, linear sweep voltammetry, staircase voltammetry, squarewave voltammetry, cyclic voltammetry, or others.

In the next step, current flow through the working electrode is measured. As described above, this step allows the presence and/or concentration of one or more redox active substances in the liquid sample to be determined.

In some embodiments, the method include determining the presence and/or number of cells in the liquid sample. By using a known number and/or concentration of cells in a liquid sample and measuring the amount of redox active substance produced under defined conditions, the relationship between cell number and amount of redox active substance can be determined. This relationship, taken together with the relationship between the voltammetric current and amount of redox active substance determined as described above, can be used to determine the relationship between voltammetric current and cell number.

In some embodiments, the method includes determining the concentration of the redox active substance by measuring the pH of the liquid sample using a pH electrode within the nanofluidic channel. The effect of pH on the voltammetric relationship between concentration of the redox active substance and current can be independently characterized. After establishing this dependence under controlled conditions, the dual inputs of voltammetric current and pH can be used to determine presence and/or concentration of the redox active substance with more accuracy and sensitivity.

## EXAMPLES

### Example 1: Materials

All bacterial tests were completed using wild-type *P. aeruginosa* strain PA14. Cell cultures were routinely grown in trypticase soy broth (TSB) (BD Biosciences 211768) at 37°C and later stored on TSB agar plates at 4°C when not in use. All amino acids were purchased from Sigma-Aldrich (St. Louis, MO) and dissolved in solution using either TSB or M63 minimal salts medium (Fisher Cat. 50-751-6740) [$(NH_4)_2SO_4$ (15 mM), $KH_2PO_4$ (100 mM)] supplemented with $MgSO_4$ (1 mM) and glycerol (0.027 mM). Electrochemical measurements were performed using commercially available Zensor TE100 (EDAQ ET077) screenprinted electrodes featuring carbon working and counter electrodes. Although the Zensor TE100 electrodes include a silver paste reference, a separate 1 M KCl Ag/AgCl reference electrode (CHI111) was employed to minimize the chances of drift in reference potential during measurements lasting several hours, which can occur when the reference electrode is in direct contact with the sample solution. All electrochemical measurements were recorded using a potentiostat (CHI842C, CH Instruments).

### Example 2: Culturing cells and measuring current

Individual amino acids were dissolved at specific concentrations in either 10 mL of TSB or M63 media. Each solution was inoculated with a specific concentration of *P. aeruginosa* and incubated at 37 C. 100 mL of each solution was removed at designated time points and loaded onto a Zensor electrode for electrochemical testing. Square-wave voltammetric scans were performed at potentials ranging from -0.4 to -0.1 V at an amplitude voltage of 0.050 V and a frequency of 15 Hz. Scans were performed for each sample three times with three replicates for each tested condition. The data was analyzed using OriginPro 9.1 (OriginLab Corporation). Baselines were created for each data set using spline interpolation with 8 base points. The resulting baseline-subtracted data set was used to determine the max currents observed from pyocyanin production.

### Example 3: Effects of specific amino acids on pyocyanin production by *P. aeruginosa*

Liquid cultures of *P. aeruginosa* were grown in either M63 minimal media or TSB to observe the effect of amino acid addition on the bacteria's production of pyocyanin using the methods described in Example 2. 10 μL of stock PA14

culture was loaded into 10 mL liquid cultures each containing one of six different amino acids (final concentration of 4 million cells per mL). To maximize the effect that individual amino acids might have on pyocyanin production, the amino acid concentrations chosen were 16-times higher than the concentrations typically found in patients infected with cystic fibrosis with the exception of tyrosine, which was only increased 4-fold due to its lower solubility (S. P. Bernier, D. G. Ha, W. Khan, J. H. Merritt and G. A. O'Toole, Res. Microbiol., 2011, 162, 680-688; and G. C. Palmer, K. L. Palmer, P. A. Jorth and M. Whiteley, J. Bacteriol., 2010, 192, 2722-2728). Electrochemical scans were taken roughly every two hours over the course of a 24-hour period.

FIGS. 9A and B show scans taken after 10 hours of growth in M63 minimal media and trypticase soy broth, respectively. A control sample with no added amino acids was also tested. Pyocyanin is expected to produce an electrochemical signal around -0.25 V vs. a Ag/AgCl reference and this result is observed for all samples grown in TSB media. However, no statistically significant difference is observed in any of the samples in M63 minimal media after 10 hours.

Consistent among all tests was that samples grown in TSB produced a pyocyanin signal faster than those grown in M63 media. Although cells can grow and divide normally in both media, TSB contains a series of additional nutrients not found in M63 minimal media, such as casein and soybean lysate, which accelerate the bacterial growth rate. However, of greater importance is that select amino acids had an up-regulatory effect on pyocyanin production as demonstrated by the samples containing tyrosine and valine. The results show that the addition of tyrosine to minimal media lowers the amount of time needed to detect the presence of *P. aeruginosa* in a sample via detection of current produced by pyocyanin. Scans were taken over the course of one day and the maximum currents are reported in FIGS. 10 A and B. The amino acids had varying effects on *P. aeruginosa's* production of pyocyanin, with tyrosine having the greatest up-regulatory effect in both media. These results link up-regulation of pyocyanin production with increased biofilm formation induced by the addition of individual amino acids (S. P. Bernier, D. G. Ha, W. Khan, J. H. Merritt and G. A. O'Toole, Res. Microbiol., 2011, 162, 680-688). Without the addition of amino acids, it took nearly 24 hours for a pyocyanin signal to be observed in M63 media, while a signal was seen within 10 hours for the control experiment using TSB. In M63 minimal media, addition of tyrosine and histidine resulted in the appearance of a pyocyanin signal in significantly less time. Addition of amino acid to TSB, however, did not appreciably change the amount of time needed to observe a pyocyanin signal. In all cases, the signal appeared 6-8 hours after the start of the experiment. However, the amount of pyocyanin produced after that point varied significantly. A small shift towards the positive potential (<0.10 V) was observed for samples grown in M63 media, which can be attributed to minor differences in the salt and pH concentration of the surrounding media. The error bars, shown in the figure, increase with time because of heterogeneity in the bacterial population. As the number of cells increases over time, they do not all divide or produce pyocyanin at exactly the same rate. This results in greater variability in the pyocyanin concentration over time, for each experiment. However, as a sensing mechanism, the primary concern is inducing the production of pyocyanin and the actual variance is secondary.

Example 4: Effects of concentration of tyrosine and valine on pyocyanin production by P. *aeruginosa*

Next, the optimal concentrations of tyrosine and valine the growth media to maximize pyocyanin production were determined. Tyrosine and valine were selected as the target amino acids to apply in the TSB media as they demonstrated the largest up-regulatory effect of the six amino acids tested. As cells grown in TSB growth media gave the fastest pyocyanin response, TSB was chosen as the growth media for the next phase of the study.

Tyrosine and valine were prepared at concentrations ranging from those quantified in typical cystic fobrosis infection levels (tyrosine: 0.2 mM, valine: 1.1 mM) to an 80-fold increase, which reached the solubility limit of the amino acids in TSB. The initial concentration of *P. aeruginosa* loaded in each sample was kept constant at roughly 4 million cells per mL and electrochemical scans were taken every two hours over the course of ten hours. FIGS. 11A and B show scans taken after eight hours of growth in addition to a control with no amino acid additives. Again, consistent among all scans was the observance of a pyocyanin peak around -0.25 V vs. a Ag/AgCl reference. Interesting to note is that the largest pyocyanin concentration for the experiments with valine was recorded for the 17.6 mM concentration and that pyocyanin production decreased when the valine concentration was raised further to 88 mM. It is possible that valine has an inhibitory effect on pyocyanin production at such high concentrations. While such inhibition has been observed for other molecules (D. J. Musk, D. A. Banko and P. J. Hergenrother, Chem. Biol., 2005, 12, 789-796), *P. aeruginosa* has not been previously studied at valine concentrations that are over 100 times greater than what is typically found in a pseudomonal infection.

FIGS. 12A and B show the increase in pyocyanin over time for each of the concentrations of tyrosine and valine tested. From the data presented in FIG. 12A, a statistically significant increase is observed in current output between six and eight hours, marking the minimum amount of time necessary to electrochemically detect a *P. aeruginosa* infection in a processed sample. Importantly, varying the concentration of tyrosine and valine added to the solution has a minimal effect on the amount of time needed for cells to up-regulate pyocyanin production, but has a significant effect on the

amounts produced after this critical time point. The rate of pyocyanin production by the cells in 16 mM tyrosine and 17.6 mM valine is nearly identical.

Example 5: Effects of *P. aeruginosa* cell concentration on pyocyanin production

The next set of tests studied how varying the initial *P. aeruginosa* cell concentration would affect the production of pyocyanin in the presence of amino acids. TSB media was used as the growth media to which tyrosine and valine were added. Tyrosine (16 mM) and valine (17.6 mM) concentrations were held constant while varying amounts of *P. aeruginosa* were added into the samples (4, 20, 40, 400 million cells per mL). Two control experiments were also included: one with an initial *P. aeruginosa* concentration of 4 million cells per mL without additional amino acids and the second with the amino acids added but no bacteria. Electrochemical scans were taken every two hours over the course of ten hours. FIGS. 13 A and B show scans taken after eight hours of growth. There is a clear correlation between the increasing starting concentration of bacteria and the amount of pyocyanin produced over a constant amount of time. For initial cell concentrations above 4 million cells per mL, 16 mM tyrosine causes *P. aeruginosa* to produce more pyocyanin than 17.6 mM valine. The minimum incubation time needed to detect a *P. aeruginosa* infection increases with decreasing initial cell concentration (FIGS. 14A and B). From the data presented in FIG. 14 A, a statistically significant increase in current output is obtained for the highest initial cell concentration (400 million cells per mL) between four and six hours after the start of the experiment. These results indicate that the amount of time necessary to detect a current change due to pyocyanin production can be used to quantify the number of initial cells present in the sample.

The theoretical cellular limit of detection for our approach, without sample preconcentration, is 0.1 cells per mL. Since a 10 mL sample volume is used in the analysis, there would be one cell in the sample in this case. It would take approximately one day to observe a pyocyanin peak for this minimal concentration. Previous work, where fresh growth media was inoculated with only a few cells from a *P. aeruginosa* colony on a plate, showed that it took approximately 24 hours to produce a 5 mM concentration of pyocyanin (T. A. Webster and E. D. Goluch, Lab Chip, 2012, 12, 5195-5201), which supports our analysis.

Example 6: Detection of pyocyanin in bodily fluids

Square-wave voltammetry was used to analyze whether pyocyanin could be detected in various bodily fluids. Human samples of urine, sputum, bronchial lavage, and whole blood with heparin, pooled from 20 healthy patients, were purchased in 1 mL aliquots from Bioreclamation. With the exception of the whole blood samples, all biological fluids were stored at -20 °C. Samples were thawed in a water bath prior to experiments. Whole blood samples were stored at 4 °C refrigerator to avoid cellular lysis. A dilution series from 1 to 100 μM was prepared by adding the appropriate volume of stock pyocyanin solution (500 μM) to 500 μL of each human biofluid sample.

*P. aeruginosa* PA14 were grown in 3 mL of TSB media for 24 hours at 37 °C until they reached stationary growth phase (~5 x $10^7$ cells/mL). The cells were spun down in a centrifuge and resuspended in 3 mL of fresh TSB media just before being spiked into biological fluids. 500 μL of each biological fluid was spiked with 150 μL of the *P. aeruginosa* PA14 media and cultured at 37 °C. 150 μL of each spiked biological fluid was removed daily and placed onto a Zensor electrode. The samples were scanned from -0.5 to 0 volts using SWV at a frequency of 15 Hz and an amplitude voltage of 50 mV. No electrolytes were added to the samples as they naturally contain a significant amount of sodium, potassium, and chloride ions. Each sample was measured three times with three different sensors for a total of nine measurements.

The pyocyanin signal from *P. aeruginosa* was readily detected in bronchial lavage (FIG 15A), sputum (FIG 15B), urine (FIG 15C), whole blood (FIG 15D), and whole blood supplemented with sodium heparin (FIG 15E) samples, although the potential that gave the peak current from pyocyanin peaked varied from about -0.32 V to -0.2 V.

Example 7: Specificity of detection of *P. aeruginosa*

The square-wave voltammetry signal due to pyocyanin can be used to specifically identify *P. aeruginosa.* Other species of infectious bacteria were tested for the ability to secrete compounds that produce an electrochemical signal similar to the one due to pyocyanin. The following bacterial strains were analyzed: *P. aeruginosa* strain PA01 transformed to express either CFP or YFP, E. coli strain K12 transformed to express either GFP or YFP, *S. aureus* strain 25923 (obtained from the American Type Culture Collection), *S. epidermis* strain 35984 (from ATCC), and a *B. cereus* strain (obtained from Yunrong Chai, Department of Biology, Northeastern University). Cells were cultured in either TSB or Lysogeny broth and analyzed as described above.

The current peak at about -0.25 V was apparent in *P. aeruginosa* cultures but not in cultures of any of the other bacterial species grown in TSB (FIG. 16A). Similar results were observed when bacteria were cultured in Lysogeny Broth (FIG. 16B).

REFERENCES

S. P. Bernier, D. G. Ha, W. Khan, J. H. Merritt and G. A. O'Toole, Res. Microbiol., 2011, 162, 680-688.

Czerwinski and Marassi, 1992, J Electroanaly Chem Interfacial Electrochem, 322, 373-381.

Czerwinski et al., 1991 J Electroanal Chem Interfacial Electrochem, 316, 211-221.

Czerwinski et al., 1995, J Electroanal Chem Interfacial Electrochem, 386, 207-211.

Dam et al., 2007, Analyst 132, 365-370; Pihel et al., 1995, Anal Chem 67, 4514-4521.

L. E. Dietrich, A. Price-Whelan, A. Petersen, M. Whiteley and D. K. Newman, Mol. Microbiol., 2006, 61, 1308-1321.

H. Fazeli, R. Akbari, S. Moghim, T. Narimani, M. R. Arabestani and A. R. Ghoddousi, J. Res. Med. Sci., 2012, 17, 332-337.

Goluch, Edgar, D., and Webster, T., WO2014015333.

Goluch et al. 2009, Anal Bioanal Chem 394, 447-456.

N. Grossowicz, P. Hayat and Y. S. Halpern, J. Gen. Microbiol., 1957, 16, 576-583.

W. He, C. Li and C. D. Lu, J. Bacteriol., 2011, 193, 2107-2115.

E. Hellinger, J. Gen. Microbiol., 1951, 5, 633-639.

R. C. Hider and X. Kong, 2010, Nat. Prod. Rep. 27: 637-657.

E. Kipnis, T. Sawa and J. Wiener-Kronish, Med. Mal. Infect., 2006, 36, 78-91.

Lacher et al., 2001, Electrophoresis 22, 2526-2536.

G. W. Lau, D. J. Hassett, H. Ran and F. Kong, Trends Mol. Med., 2004, 10, 599-606.

T. W. Lee, K. G. Brownlee, M. Denton, J. M. Littlewood and S. P. Conway, Pediatr. Pulmonol., 2004, 37, 104-110.

J. B. Lyczak, C. L. Cannon and G. B. Pier, Clin. Microbiol. Rev., 2002, 15, 194-222.

J. B. Lyczak, C. L. Cannon and G. B. Pier, Microbes Infect., 2000, 2, 1051-1060.

D. J. Musk, D. A. Banko and P. J. Hergenrother, Chem. Biol., 2005, 12, 789-796.

Odijk et al., 2009, Lab Chip, 9, 1687-1693.

G. C. Palmer, K. L. Palmer, P. A. Jorth and M. Whiteley, J. Bacteriol., 2010, 192, 2722-2728.

L. S. Pierson, 3rd and E. A. Pierson, Appl. Microbiol. Biotechnol., 2010, 86, 1659-1670.

F. Ratjen and G. Doring, Lancet, 2003, 361, 681-689.

D. Sharp, P. Gladstone, R. B. Smith, S. Forsythe and J. Davis, Bioelectrochemistry, 2010, 77, 114-119.

Straver, et al., Lab Chip 12, 1548-1553.

V. H. Tam, K. T. Chang, K. Abdelraouf, C. G. Brioso, M. Ameka, L. A. McCaskey, J. S. Weston, J. P. Caeiro and

K. W. Garey, Antimicrob. Agents Chemother., 2010, 54, 1160-1164.

S. R. Thomas, A. Ray, M. E. Hodson and T. L. Pitt, Thorax, 2000, 55, 795-797.

V. B. Wang, S. L. Chua, B. Cao, T. Seviour, V. J. Nesatyy, E. Marsili, S. Kjelleberg, M. Givskov, T. Tolker-Nielsen, H. Song, J. S. Loo and L. Yang, PLoS One, 2013, 8, e63129.

T. A. Webster and E. D. Goluch, Lab Chip, 2012, 12, 5195-5201.

T. A. Webster, H. J. Sismaet, J. L. Conte, I. P. Chan and E. D. Goluch, Biosens. Bioelectron., 2014, 60, 265-270.

T. A. Webster, H. J. Sismaet and E. D. Goluch, Nano LIFE, 2013, 03, 1340011.

Wolfrum, et al., 2008 Anal Chem 80, 972-977.

Zeng et al., 2011, Nano Lett, 11, 262-268.

Zevenbergen et al., 2007, Nano Lett 7, 384-388).

Zevenbergen et al., 2011, Nano Lett 11, 2881-2886.

## Claims

**1.** A nanofluidic electrode assembly for detecting a redox active substance (400) secreted by a cell in a liquid sample, the nanofluidic electrode assembly comprising:

(a) a nanofluidic channel (380) disposed in a substrate (320);
(b) an inlet and an outlet port (370) in fluid connection with the nanofluidic channel, the inlet and outlet ports configured to add and/or remove liquid from the liquid sample to or from the nanofluidic channel;
(c) a first working electrode (310, 510) disposed in the nanofluidic channel, wherein the first working electrode is suitable for applying a potential sufficient to oxidize the redox active substance;
(d) a second working electrode (340, 520) disposed in the nanofluidic channel, wherein the second working electrode is suitable for applying a potential sufficient to reduce the redox active substance;
(e) a reference electrode (350) disposed in the nanofluidic channel; and
(f) a matrix (390) comprising a chemical agent that stimulates secretion of the redox active substance by the cell, wherein the matrix is capable of releasing the chemical agent, and wherein the matrix is disposed in one or more of the following sites:

(1) in the nanofluidic channel;
(2) in the inlet and/or outlet port;
(3) on at least a portion of a surface of the first working electrode;
(4) on at least a portion of a surface of the second working electrode;
(5) on at least a portion of a surface of the reference electrode; and
(6) on at least a portion of a surface of the substrate, wherein the surface of the substrate is adjacent to the inlet and/or outlet port;

wherein the concentration of the redox active substance is detected as current flow through the working electrodes.

**2.** A device comprising the nanofluidic electrode assembly of claim 1 and a counter electrode in fluid connection with the nanofluidic channel.

**3.** The nanofluidic electrode assembly of claim 1, wherein the chemical agent is an amino acid, such as tyrosine or valine.

**4.** The nanofluidic electrode assembly of claim 1, wherein the matrix comprises gelatin, chitosan, alginate, fibrin, collagen, elastin, agar, agarose, hyaluronic acid, dextran, cellulose, poly(vinyl alcohol), polyacrylamide, poly(N-vinylpyrolidone), poly(hydroxyethyl methacrylate), poly(ethylene oxide), poly(ethylene glycol), poly(ethylene glycol)

monomethyl ether, poly(acrylate), polymethacrylate, poly(methylacrylate), poly(methyl methacrylate) or poly(lactic acid).

**5.** The nanofluidic electrode assembly of claim 1, wherein the redox active substance is a siderophore, such as pyocyanin.

**6.** The nanofluidic electrode assembly of claim 1, wherein the reference electrode, and/or the first working electrode, and/or the second working electrode, forms at least a portion of a wall of the nanofluidic channel.

**7.** The nanofluidic electrode assembly of claim 1, wherein at least a portion of a surface of the substrate is coated with a coating that promotes adhesion of the cell to the nanofluidic electrode assembly.

**8.** A device comprising the nanofluidic electrode assembly of claim 1 and a processor capable of performing data analysis using the current flow through the working electrodes.

**9.** A device for simultaneous detection of a plurality of redox active substances secreted by one or more types of cells, the device comprising a plurality of the nanofluidic electrode assemblies of claim 1.

**10.** A device comprising:

(a) at least one of the nanofluidic assemblies of claim 1; and
(b) a microfluidic channel (1770) capable of accepting the liquid sample, the microfluidic channel in fluid connection with the nanofluidic channel via the inlet and outlet ports (1800, 1810).

**11.** The device of claim 10, wherein the matrix is disposed in the microfluidic channel adjacent to the inlet port and/or outlet port.

**12.** The device of claim10, wherein the dimensions of the nanofluidic channel exclude passage of the cell from the microfluidic channel into the nanofluidic channel

**13.** A method of detecting a redox active substance secreted by a cell in a liquid sample, the method comprising:

(a) providing a nanofluidic electrode assembly of claim 1;
(b) exposing the liquid sample to the matrix, wherein the chemical agent is released from the matrix;
(c) charging the reference electrode to stabilize its potential;
(d) applying a potential suitable for oxidizing the redox active substance to the first working electrode and applying a potential suitable for reducing the redox active substance to the second working electrode, wherein the redox active substance has a halfway potential value between the potential of the first working electrode and the potential of the second working electrode;
(e) measuring current flow through the first and the second working electrodes;

wherein a current flow above a threshold value indicates a presence of the redox active substance, and a current flow below the threshold value indicates an absence of the redox active substance.

**14.** The method of claim 13, wherein detection of the presence of the redox active substance indicates a presence of a cell that secretes the redox active substance, and detection of the absence of the redox active substance indicates an absence of the cell that secretes the redox active substance.

**15.** A method of simultaneously detecting a first and second redox active substances secreted by one or more types of cells in a liquid sample, wherein each of the first and second redox active substances has a distinct redox potential, the method comprising:

(a) providing a nanofluidic assembly of claim 1;
(b) exposing the liquid sample to the matrix, wherein one or more chemical agents are released from the matrix;
(c) charging the reference electrode to stabilize its potential;
(d) applying a first set of oxidizing potentials suitable for oxidizing the first redox active substance and a second set of oxidizing potentials suitable for oxidizing the second redox active substance at the first working electrode, and applying a first set of reducing potentials suitable for reducing the first redox active substance and a second

set of reducing potentials suitable for reducing the second redox active substance at the second working electrode; and
(e) measuring current flow through the first and the second working electrodes;

wherein a current flow above a first threshold value during application of the first sets of oxidizing and reducing potentials indicates a presence of the first redox active substance, and a current flow below the first threshold value indicates an absence of the first redox active substance; and wherein a current flow above a second threshold value during application of the second sets of oxidizing and reducing potentials indicates a presence of the second redox active substance, and a current flow below the second threshold value indicates an absence of the second redox active substance.

**16.** The method any one of claims 13 to 15, wherein the liquid sample is a sample from a patient with cystic fibrosis.

## Patentansprüche

**1.** Nanofluid-Elektrodenanordnung zum Erkennen einer redoxaktiven Substanz (400), die durch eine Zelle in einer flüssigen Probe sekretiert wurde, wobei die Nanofluid-Elektrodenanordnung umfasst:

(a) einen Nanofluid-Kanal (380), der in einem Substrat (320) angeordnet ist;
(b) eine Einlass- und eine Auslassöffnung (370) in Fluid-Verbindung mit dem Nanofluid-Kanal, wobei die Einlass- und Auslassöffnung eingerichtet sind, um Flüssigkeit aus der Probe zum Nanofluid-Kanal hinzuzufügen und/oder daraus zu entfernen;
(c) eine im Nanofluid-Kanal angeordnete erste Arbeitselektrode (310, 510), wobei die erste Arbeitselektrode zum Anlegen eines Potenzials geeignet ist, das ausreicht, um die redoxaktive Substanz zu oxidieren;
(d) eine im Nanofluid-Kanal angeordnete zweite Arbeitselektrode (340, 520), wobei die zweite Arbeitselektrode zum Anlegen eines Potenzials geeignet ist, das ausreicht, um die redoxaktive Substanz zu reduzieren;
(e) eine im Nanofluid-Kanal angeordnete Referenzelektrode (350); und
(f) eine Matrix (390), umfassend einen chemischen Wirkstoff, der die Sekretion der redoxaktiven Substanz durch die Zelle stimuliert, wobei die Matrix in der Lage ist, den chemischen Wirkstoff freizusetzen, und wobei die Matrix an einer oder mehreren der folgenden Stellen angeordnet ist:

(1) im Nanofluid-Kanal;
(2) in der Einlass- und/oder Auslassöffnung;
(3) an mindestens einem Abschnitt einer Oberfläche der ersten Arbeitselektrode;
(4) an mindestens einem Abschnitt einer Oberfläche der zweiten Arbeitselektrode;
(5) an mindestens einem Abschnitt einer Oberfläche der Referenzelektrode; und
(6) an mindestens einem Abschnitt einer Oberfläche des Substrats, wobei die Oberfläche des Substrats der Einlass- und/oder Auslassöffnung benachbart ist;

wobei die Konzentration der redoxaktiven Substanz als Stromfluss durch die Arbeitselektroden erkannt wird.

**2.** Vorrichtung, umfassend die Nanofluid-Elektrodenanordnung nach Anspruch 1 und eine Zählerelektrode in Fluid-Verbindung mit dem Nanofluid-Kanal.

**3.** Nanofluid-Elektrodenanordnung nach Anspruch 1, wobei der chemische Wirkstoff eine Aminosäure wie z. B. Tyrosin oder Valin ist.

**4.** Nanofluid-Elektrodenanordnung nach Anspruch 1, wobei die Matrix Gelatine, Chitosan, Alginat, Fibrin, Collagen, Elastin, Agar, Agarose, Hyaluronsäure, Dextran, Cellulose, Poly-(Vinylalkohol), Polyacrylamid, Poly-(N-Vinylpyrolidon), Poly-(Hydroxyethylmethacrylat), Poly-(Ethylenoxid), Poly-(Ethylenglycol), Poly-(Ethylenglycol) Monomethylether, Poly-(Acrylat), Polymethacrylat, Poly-(Methylacrylat), Poly-(Methylmethacrylat) oder Poly-(Milchsäure) umfasst.

**5.** Nanofluid-Elektrodenanordnung nach Anspruch 1, wobei die redoxaktive Substanz ein Siderophor wie z. B. Pyocyanin ist.

**6.** Nanofluid-Elektrodenanordnung nach Anspruch 1, wobei die Referenzelektrode und/oder die erste Arbeitselektrode

und/oder die zweite Arbeitselektrode mindestens einen Abschnitt einer Wand des Nanofluid-Kanals bilden.

**7.** Nanofluid-Elektrodenanordnung nach Anspruch 1, wobei mindestens ein Abschnitt einer Oberfläche des Substrats mit einer Beschichtung beschichtet ist, die die Adhäsion der Zelle an der Nanofluid-Elektrodenanordnung fördert.

**8.** Vorrichtung, umfassend die Nanofluid-Elektrodenanordnung nach Anspruch 1 und einen Prozessor, der in der Lage ist, unter Verwendung des Stromflusses durch die Arbeitselektroden eine Datenanalyse durchzuführen.

**9.** Vorrichtung zur gleichzeitigen Erkennung einer Vielzahl redoxaktiver Substanzen, die durch eine oder mehrere Arten von Zellen sekretiert wurden, wobei die Vorrichtung eine Vielzahl der Nanofluid-Elektrodenanordnungen nach Anspruch 1 umfasst.

**10.** Vorrichtung, umfassend:

(a) mindestens eine der Nanofluid-Anordnungen nach Anspruch 1; und
(b) einen Mikrofluid-Kanal (1770), der in der Lage ist, die flüssige Probe aufzunehmen, wobei der Mikrofluid-Kanal über die Einlass- und Auslassöffnung (1800, 1810) in Fluid-Verbindung mit dem Nanofluid-Kanal steht.

**11.** Vorrichtung nach Anspruch 10, wobei die Matrix im Mikrofluid-Kanal benachbart zur Einlassöffnung und/oder Auslassöffnung angeordnet ist.

**12.** Vorrichtung nach Anspruch 10, wobei die Abmessungen des Nanofluid-Kanals ausschließen, dass die Zelle aus dem Mikrofluid-Kanal in den Nanofluid-Kanal gelangt.

**13.** Verfahren zum Erkennen einer redoxaktiven Substanz, die durch eine Zelle in einer flüssigen Probe sekretiert wurde, wobei das Verfahren umfasst:

(a) Bereitstellen einer Nanofluid-Elektrodenanordnung nach Anspruch 1;
(b) Aussetzen der flüssigen Probe gegenüber der Matrix, wobei der chemische Wirkstoff aus der Matrix freigesetzt wird;
(c) Laden der Referenzelektrode zur Stabilisierung ihres Potenzials;
(d) Anlegen eines zum Oxidieren der redoxaktiven Substanz geeigneten Potenzials an die erste Arbeitselektrode und Anlegen eines zum Reduzieren der redoxaktiven Substanz geeigneten Potenzials an die zweite Arbeitselektrode, wobei die redoxaktive Substanz einen Potenzialwert aufweist, der der Hälfte zwischen dem Potenzial der ersten Arbeitselektrode und dem Potenzial der zweiten Arbeitselektrode entspricht;
(e) Messen des Stromflusses durch die erste und die zweite Arbeitselektrode;

wobei ein Stromfluss oberhalb eines Schwellenwerts eine Anwesenheit der redoxaktiven Substanz anzeigt und ein Stromfluss unterhalb des Schwellenwerts eine Abwesenheit der redoxaktiven Substanz anzeigt.

**14.** Verfahren nach Anspruch 13, wobei die Erkennung der Anwesenheit der redoxaktiven Substanz eine Anwesenheit einer Zelle anzeigt, die die redoxaktive Substanz sekretiert, und die Erkennung der Abwesenheit der redoxaktiven Substanz eine Abwesenheit der Zelle anzeigt, die die redoxaktive Substanz sekretiert.

**15.** Verfahren zum gleichzeitigen Erkennen einer ersten und zweiten redoxaktiven Substanz, die durch eine oder mehrere Arten von Zellen in einer flüssigen Probe sekretiert wurden, wobei jede der ersten und zweiten redoxaktiven Substanz ein voneinander unterschiedliches Redoxpotenzial aufweist, wobei das Verfahren umfasst:

(a) Bereitstellen einer Nanofluid-Anordnung nach Anspruch 1;
(b) Aussetzen der flüssigen Probe gegenüber der Matrix, wobei ein oder mehrere chemische Wirkstoffe aus der Matrix freigesetzt werden;
(c) Laden der Referenzelektrode zur Stabilisierung ihres Potenzials;
(d) Anlegen einer ersten Menge von Oxidationspotenzialen, die zum Oxidieren der ersten redoxaktiven Substanz geeignet sind, und einer zweiten Menge von Oxidationspotenzialen, zum Oxidieren der zweiten redoxaktiven Substanz geeignet sind, an die erste Arbeitselektrode und Anlegen einer ersten Menge von Reduktionspotenzialen, die zum Reduzieren der ersten redoxaktiven Substanz geeignet sind, und einer zweiten Menge von Reduktionspotenzialen, die zum Reduzieren der zweiten redoxaktiven Substanz geeignet sind, an die zweite Arbeitselektrode; und

(e) Messen des Stromflusses durch die erste und die zweite Arbeitselektrode; wobei ein Stromfluss oberhalb eines Schwellenwerts während des Anlegens der ersten Mengen von Oxidations- und Reduktionspotenzialen eine Anwesenheit der ersten redoxaktiven Substanz anzeigt und ein Stromfluss unterhalb des Schwellenwerts eine Abwesenheit der ersten redoxaktiven Substanz anzeigt; und wobei ein Stromfluss oberhalb eines zweiten Schwellenwerts während des Anlegens der zweiten Mengen von Oxidations- und Reduktionspotenzialen eine Anwesenheit der zweiten redoxaktiven Substanz anzeigt und ein Stromfluss unterhalb des zweiten Schwellenwerts eine Abwesenheit der zweiten redoxaktiven Substanz anzeigt.

**16.** Verfahren nach einem der Ansprüche 13 bis 15, wobei die flüssige Probe eine Probe von einem Patienten mit zystischer Fibrose ist.

**Revendications**

**1.** Ensemble électrode nanofluidique pour détecter une substance active rédox (400) sécrétée par une cellule dans un échantillon liquide, l'ensemble électrode nanofluidique comprenant :

(a) un canal nanofluidique (380) disposé dans un substrat (320) ;
(b) un orifice d'entrée et de sortie (370) en connexion fluidique avec le canal nanofluidique, les orifices d'entrée et de sortie étant configurés pour ajouter et/ou retirer un liquide à partir de l'échantillon liquide vers ou depuis le canal nanofluidique ;
(c) une première électrode de travail (310, 510) disposée dans le canal nanofluidique, dans lequel la première électrode de travail est appropriée pour appliquer un potentiel suffisant pour oxyder la substance active rédox ;
(d) une seconde électrode de travail (340, 520) disposée dans le canal nanofluidique, dans lequel la seconde électrode de travail est appropriée pour appliquer un potentiel suffisant pour réduire la substance active rédox ;
(e) une électrode de référence (350) disposée dans le canal nanofluidique ; et
(f) une matrice (390) comprenant un agent chimique qui stimule la sécrétion de la substance active rédox par la cellule, dans lequel la matrice est capable de libérer l'agent chimique, et dans lequel la matrice est disposée dans un ou plusieurs des sites suivants :

(1) dans le canal nanofluidique ;
(2) dans l'orifice d'entrée et/ou de sortie ;
(3) sur au moins une partie d'une surface de la première électrode de travail ;
(4) sur au moins une partie d'une surface de la seconde électrode de travail ;
(5) sur au moins une partie d'une surface de l'électrode de référence ; et
(6) sur au moins une partie d'une surface du substrat, dans lequel la surface du substrat est adjacente à l'orifice d'entrée et/ou de sortie ;

dans lequel la concentration de la substance active rédox est détectée sous la forme d'une circulation de courant à travers les électrodes de travail.

**2.** Dispositif comprenant l'ensemble électrode nanofluidique selon la revendication 1 et une contre-électrode en connexion fluidique avec le canal nanofluidique.

**3.** Ensemble électrode nanofluidique selon la revendication 1, dans lequel l'agent chimique est un acide aminé, tel que la tyrosine ou la valine.

**4.** Ensemble électrode nanofluidique selon la revendication 1, dans lequel la matrice comprend une gélatine, un chitosan, un alginate, une fibrine, un collagène, une élastine, une gélose, un agarose, un acide hyaluronique, un dextrane, une cellulose, un poly(alcool vinylique), un polyacrylamide, un poly(N-vinyl-pyrrolidone), un poly(méthacrylate d'hydroxyéthyle), un poly(oxyde d'éthylène), un poly(éthylène glycol), un poly(éthylène glycol) monométhyl éther, un poly(acrylate), un polyméthacrylate, un poly(acrylate de méthyle), un poly(méthacrylate de méthyle) ou un poly(acide lactique).

**5.** Ensemble électrode nanofluidique selon la revendication 1, dans lequel la substance active rédox est un sidérophore, tel que la pyocyanine.

**6.** Ensemble électrode nanofluidique selon la revendication 1, dans lequel l'électrode de référence, et/ou la première

électrode de travail, et/ou la seconde électrode de travail, forment au moins une partie d'une paroi du canal nanofluidique.

7. Ensemble électrode nanofluidique selon la revendication 1, dans lequel au moins une partie d'une surface du substrat est revêtue avec un revêtement qui favorise l'adhérence de la cellule à l'ensemble électrode nanofluidique.

8. Dispositif comprenant l'ensemble électrode nanofluidique selon la revendication 1 et un processeur capable d'effectuer une analyse de données en utilisant la circulation de courant à travers les électrodes de travail.

9. Dispositif pour la détection simultanée d'une pluralité de substances actives rédox sécrétées par un ou plusieurs types de cellules, le dispositif comprenant une pluralité des ensembles électrode nanofluidique selon la revendication 1.

10. Dispositif comprenant :

(a) au moins un des ensembles nanofluidiques selon la revendication 1 ; et
(b) un canal microfluidique (1770) capable d'accepter l'échantillon liquide, le canal microfluidique étant en connexion fluidique avec le canal nanofluidique via les orifices d'entrée et de sortie (1800, 1810).

11. Dispositif selon la revendication 10, dans lequel la matrice est disposée dans le canal microfluidique adjacent à l'orifice d'entrée et/ou l'orifice de sortie.

12. Dispositif selon la revendication 10, dans lequel les dimensions du canal nanofluidique excluent le passage de la cellule depuis le canal microfluidique dans le canal nanofluidique.

13. Procédé de détection d'une substance active rédox sécrétée par une cellule dans un échantillon liquide, le procédé comprenant :

(a) la fourniture d'un ensemble électrode nanofluidique selon la revendication 1 ;
(b) l'exposition de l'échantillon liquide à la matrice, dans lequel l'agent chimique est libéré à partir de la matrice ;
(c) la charge de l'électrode de référence pour stabiliser son potentiel ;
(d) l'application d'un potentiel approprié pour oxyder la substance active rédox à la première électrode de travail et l'application d'un potentiel approprié pour réduire la substance active rédox à la seconde électrode de travail, dans lequel la substance active rédox présente une valeur de potentiel intermédiaire entre le potentiel de la première électrode de travail et le potentiel de la seconde électrode de travail ;
(e) la mesure d'une circulation de courant à travers les première et seconde électrodes de travail ; dans lequel une circulation de courant au-dessus d'une valeur seuil indique une présence de la substance active rédox, et une circulation de courant en-dessous de la valeur seuil indique une absence de la substance active rédox.

14. Procédé selon la revendication 13, dans lequel la détection de la présence de la substance active rédox indique une présence d'une cellule qui sécrète la substance active rédox, et la détection de l'absence de la substance active rédox indique une absence de la cellule qui sécrète la substance active rédox.

15. Procédé de détection simultanée d'une première et d'une seconde substance active rédox sécrétées par un ou plusieurs types de cellules dans un échantillon liquide, dans lequel chacune des première et seconde substances actives rédox présente un potentiel rédox distinct, le procédé comprenant :

(a) la fourniture d'un ensemble nanofluidique selon la revendication 1 ;
(b) l'exposition de l'échantillon liquide à la matrice, dans lequel un ou plusieurs agents chimiques sont libérés à partir de la matrice ;
(c) la charge de l'électrode de référence pour stabiliser son potentiel ;
(d) l'application d'un premier ensemble de potentiels d'oxydation appropriés pour oxyder la première substance active rédox et d'un second ensemble de potentiels d'oxydation appropriés pour oxyder la seconde substance active rédox au niveau de la première électrode de travail, et l'application d'un premier ensemble de potentiels de réduction appropriés pour réduire la première substance active rédox et d'un second ensemble de potentiels de réduction appropriés pour réduire la seconde substance active rédox au niveau de la seconde électrode de travail ; et
(e) la mesure d'une circulation de courant à travers les première et seconde électrodes de travail ; dans lequel

une circulation de courant au-dessus d'une première valeur seuil lors de l'application des premiers ensembles de potentiels d'oxydation et de réduction indique une présence de la première substance active rédox, et une circulation de courant en-dessous de la première valeur seuil indique une absence de la première substance active rédox ; et dans lequel une circulation de courant au-dessus d'une seconde valeur seuil lors de l'application des seconds ensembles de potentiels d'oxydation et de réduction indique une présence de la seconde substance active rédox, et une circulation de courant en-dessous de la seconde valeur seuil indique une absence de la seconde substance active rédox.

**16.** Procédé selon une quelconque des revendications 13 à 15, dans lequel l'échantillon liquide est un échantillon provenant d'un patient souffrant d'une fibrose kystique.

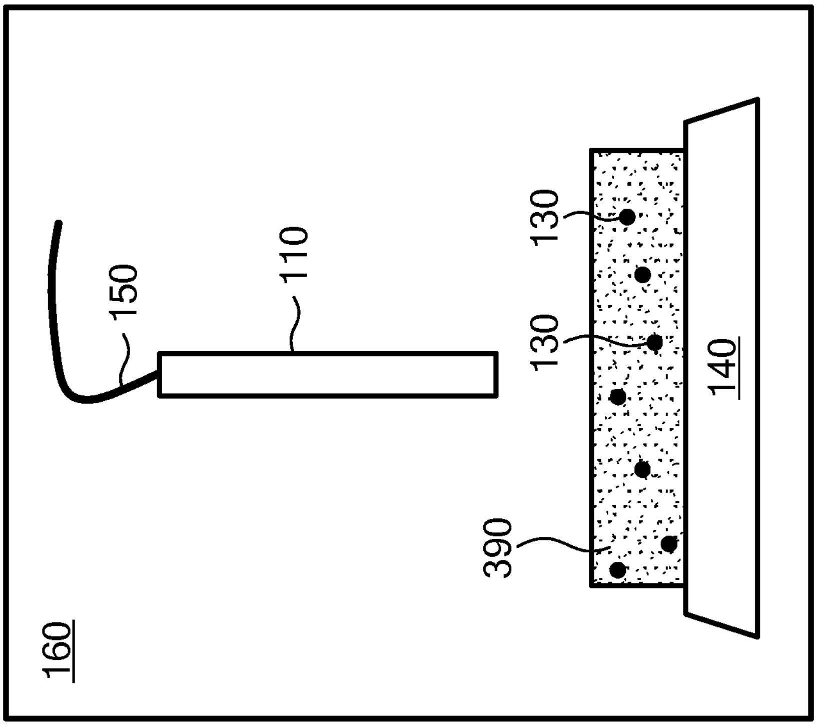


FIG. 1

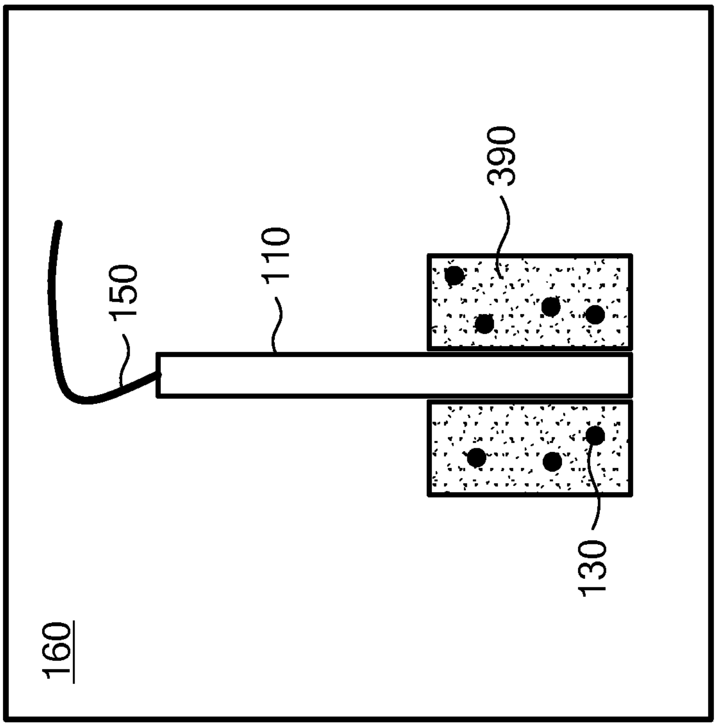


FIG. 2

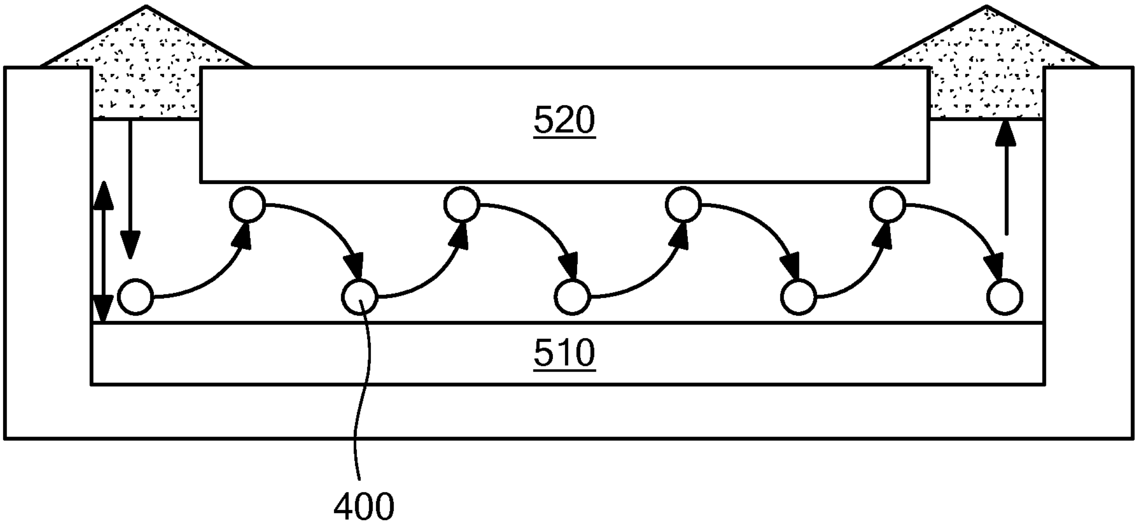
520
510
400

FIG. 3

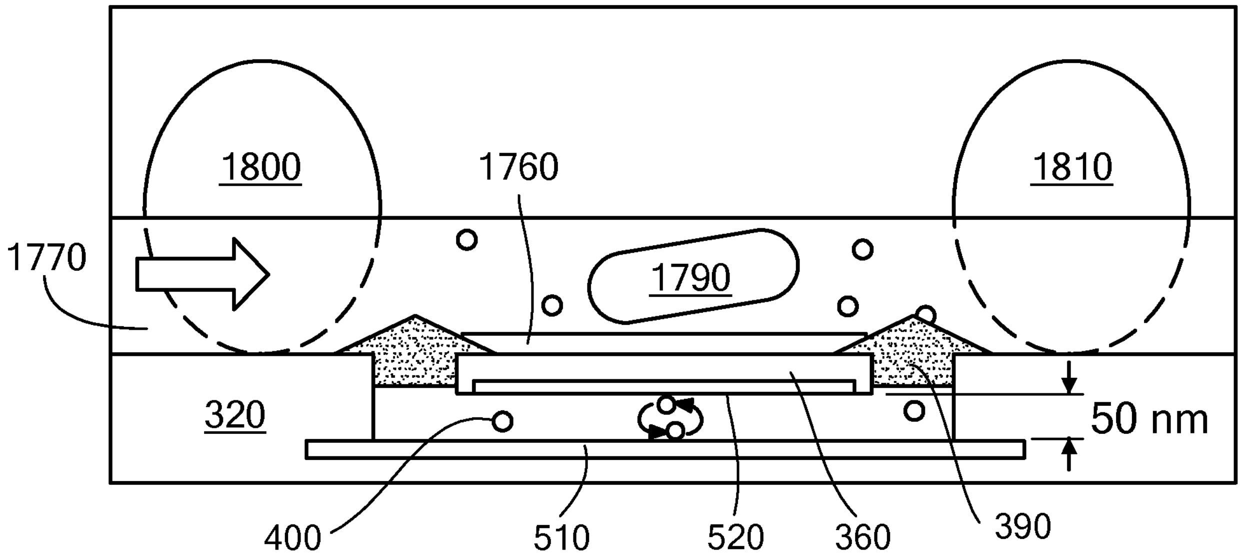
1800
1760
1810
1770
1790
320
50 nm
400
510
520
360
390

FIG. 4

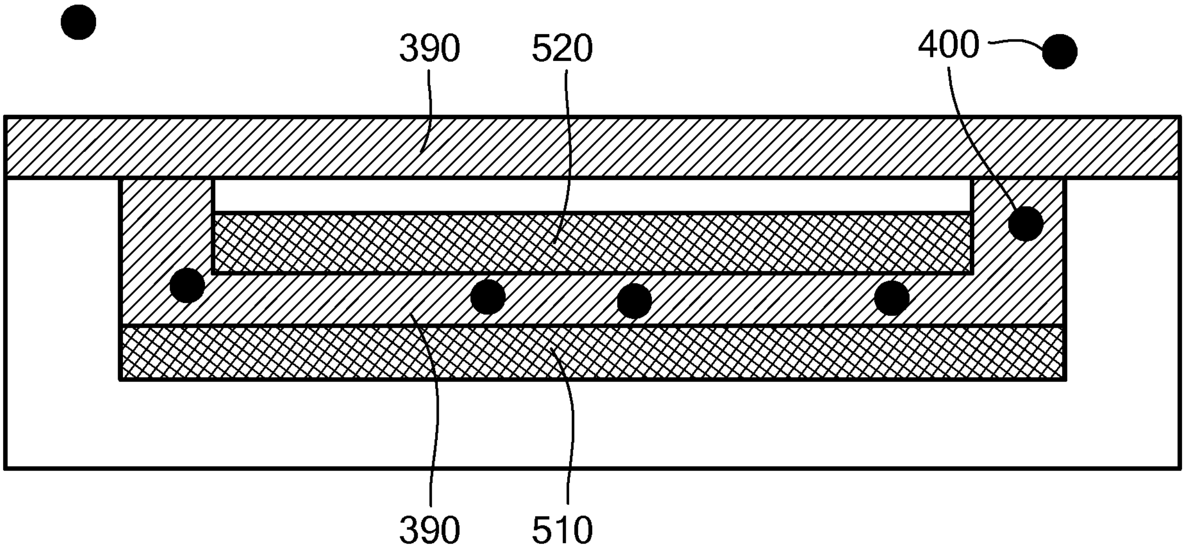


FIG. 5

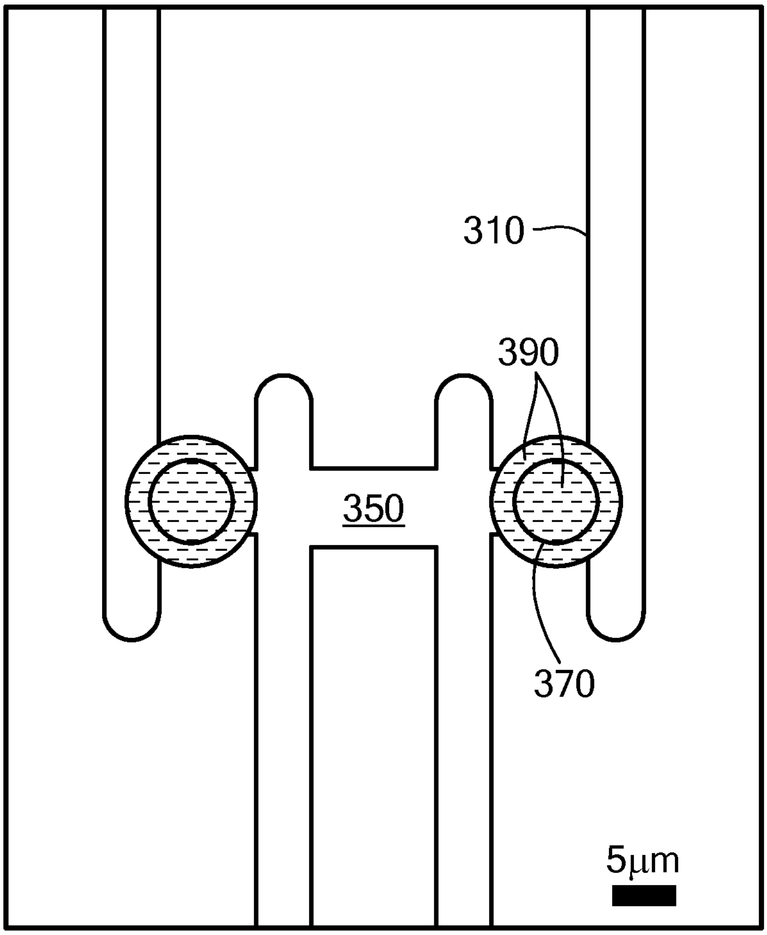


FIG. 6

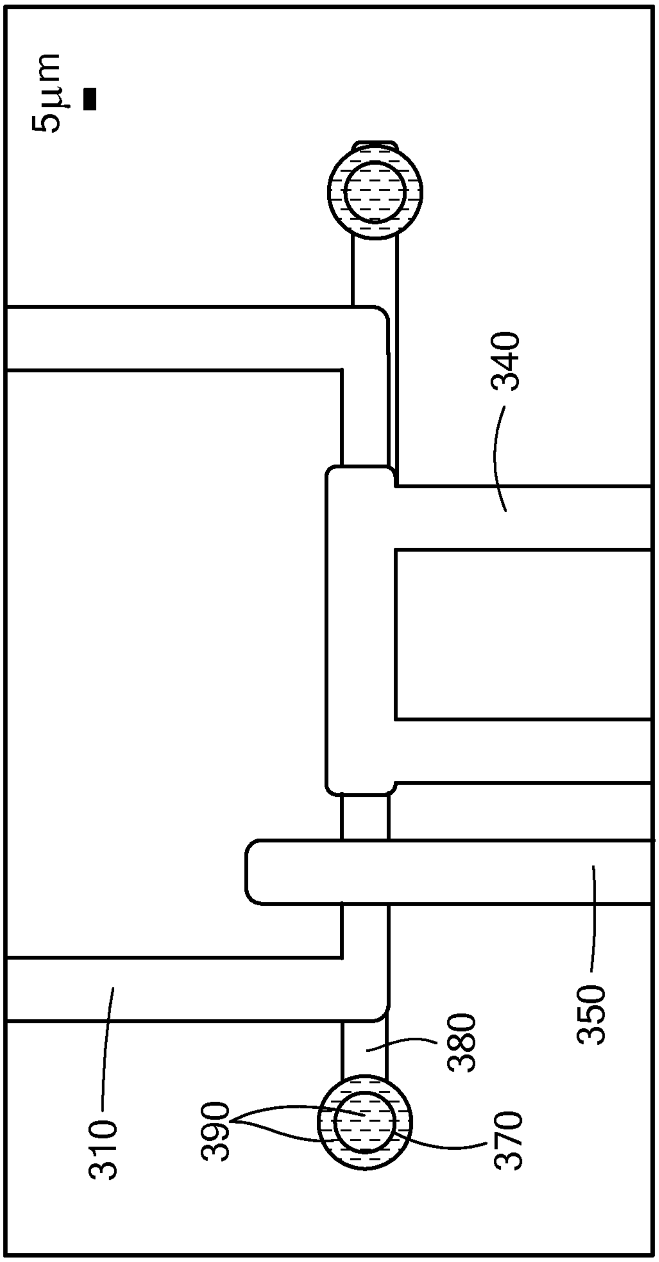


*FIG. 7*

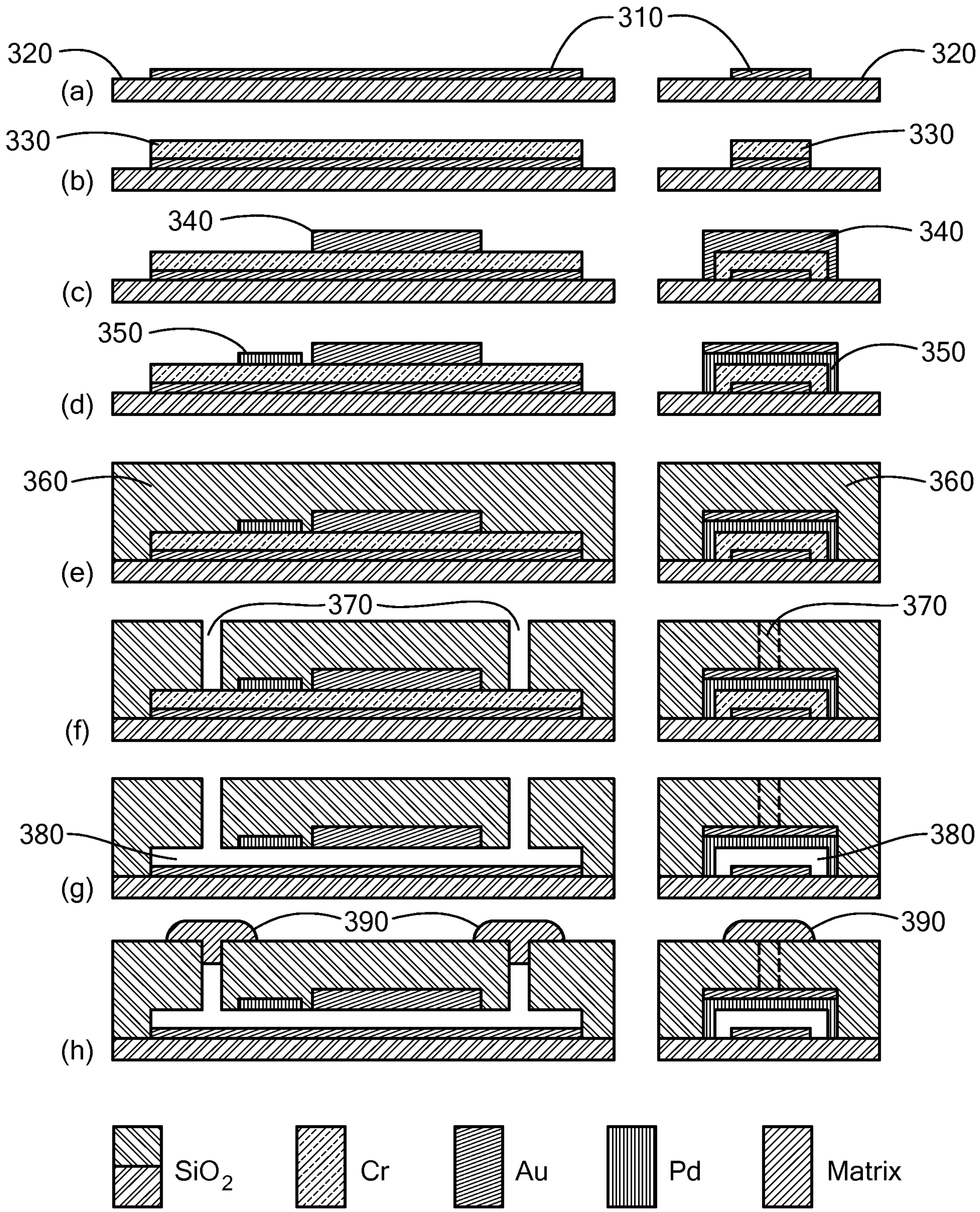
310
320
320
(a)
330
330
(b)
340
340
(c)
350
350
(d)
360
360
(e)
370
370
(f)
380
380
(g)
390
390
(h)
SiO2
Cr
Au
Pd
Matrix

*FIG. 8*

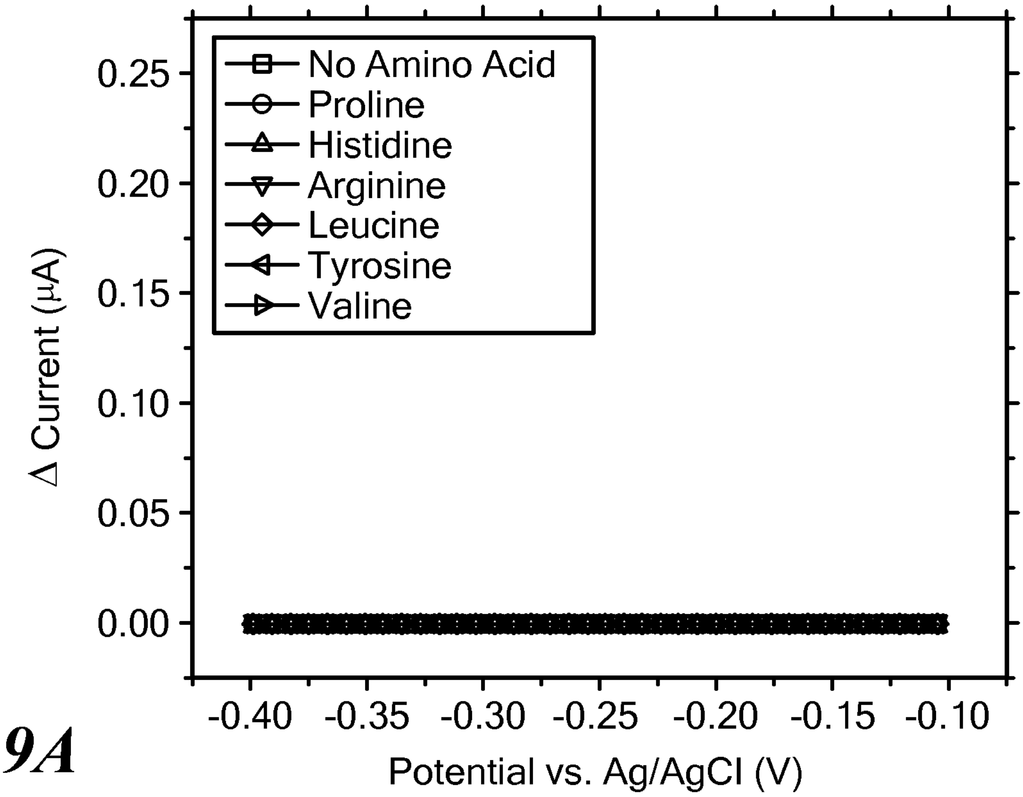

0.25
0.20
0.15
0.10
0.05
0.00
Δ Current (μA)
-0.40 -0.35 -0.30 -0.25 -0.20 -0.15 -0.10
Potential vs. Ag/AgCl (V)
No Amino Acid
Proline
Histidine
Arginine
Leucine
Tyrosine
Valine


*FIG. 9A*

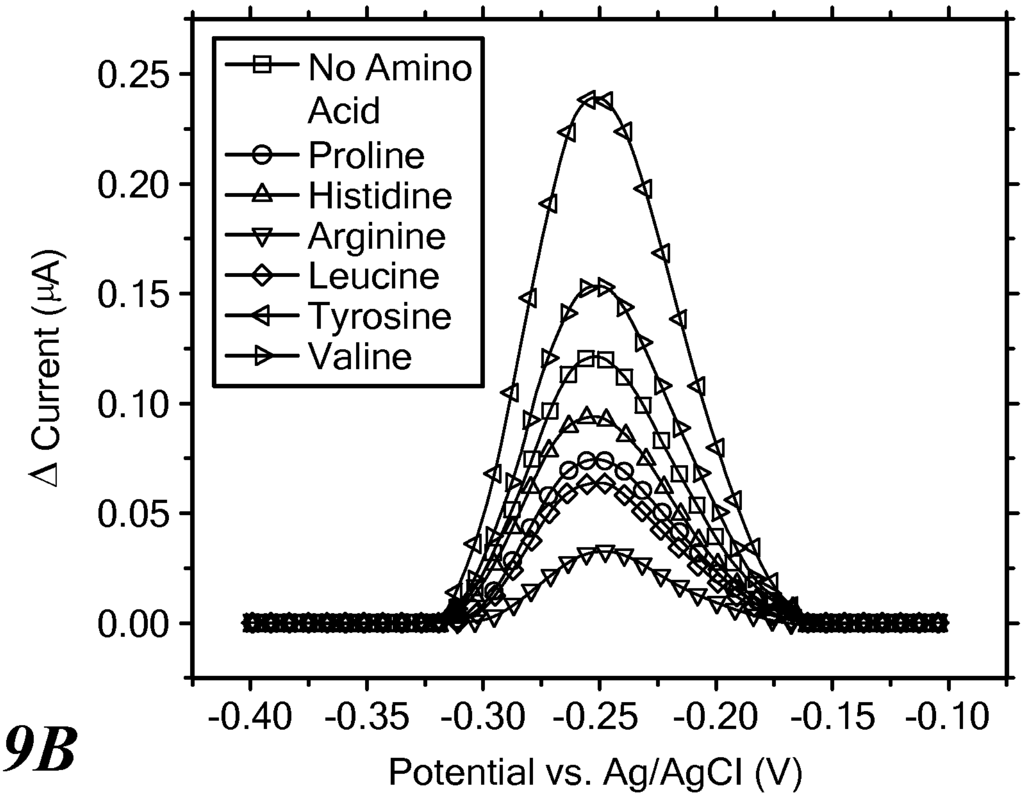

0.25
0.20
0.15
0.10
0.05
0.00
Δ Current (μA)
-0.40 -0.35 -0.30 -0.25 -0.20 -0.15 -0.10
Potential vs. Ag/AgCl (V)
No Amino Acid
Proline
Histidine
Arginine
Leucine
Tyrosine
Valine


*FIG. 9B*

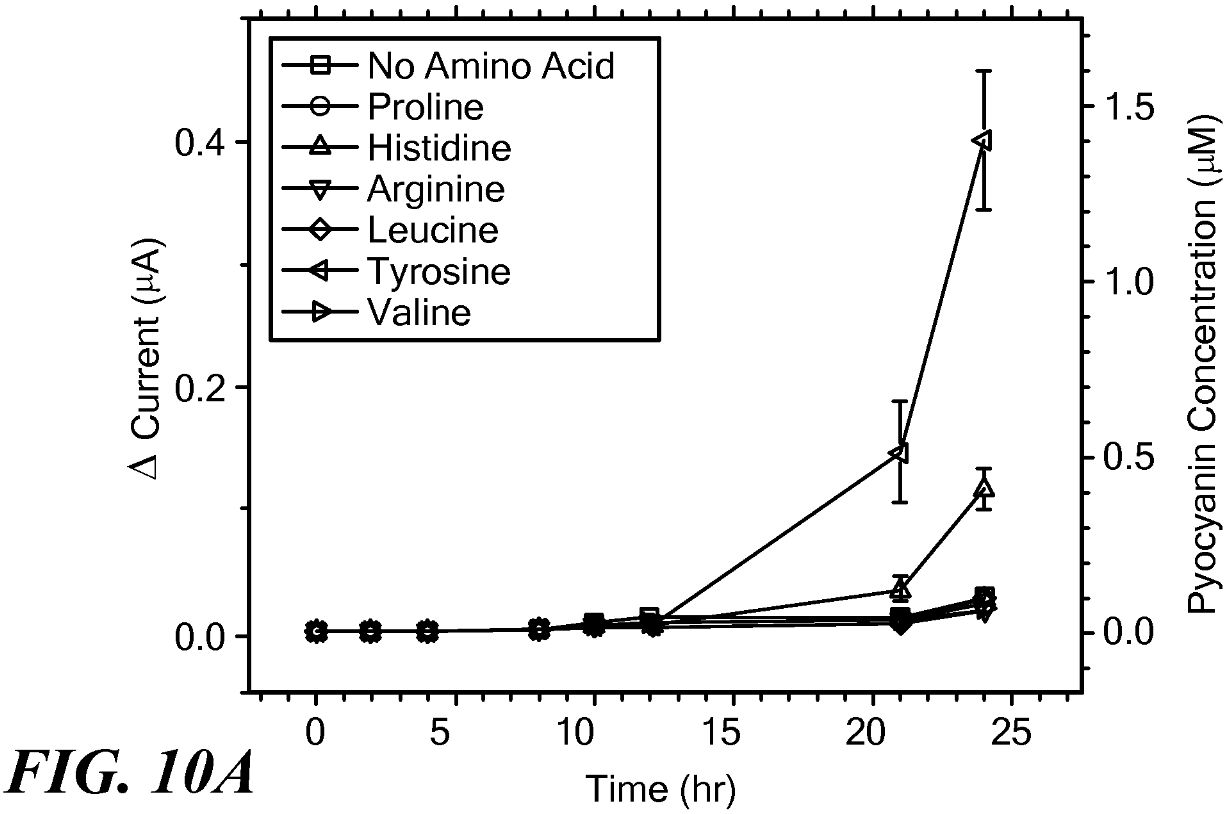


FIG. 10A

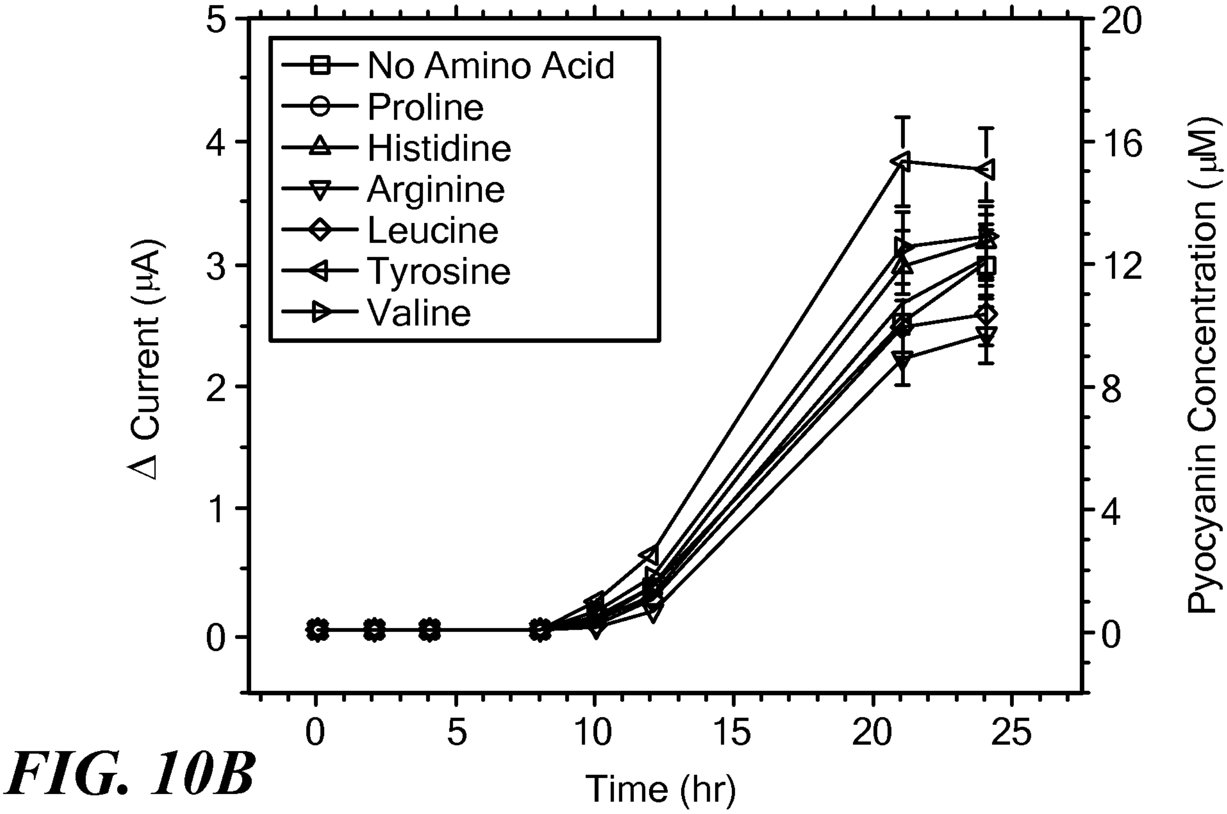


FIG. 10B

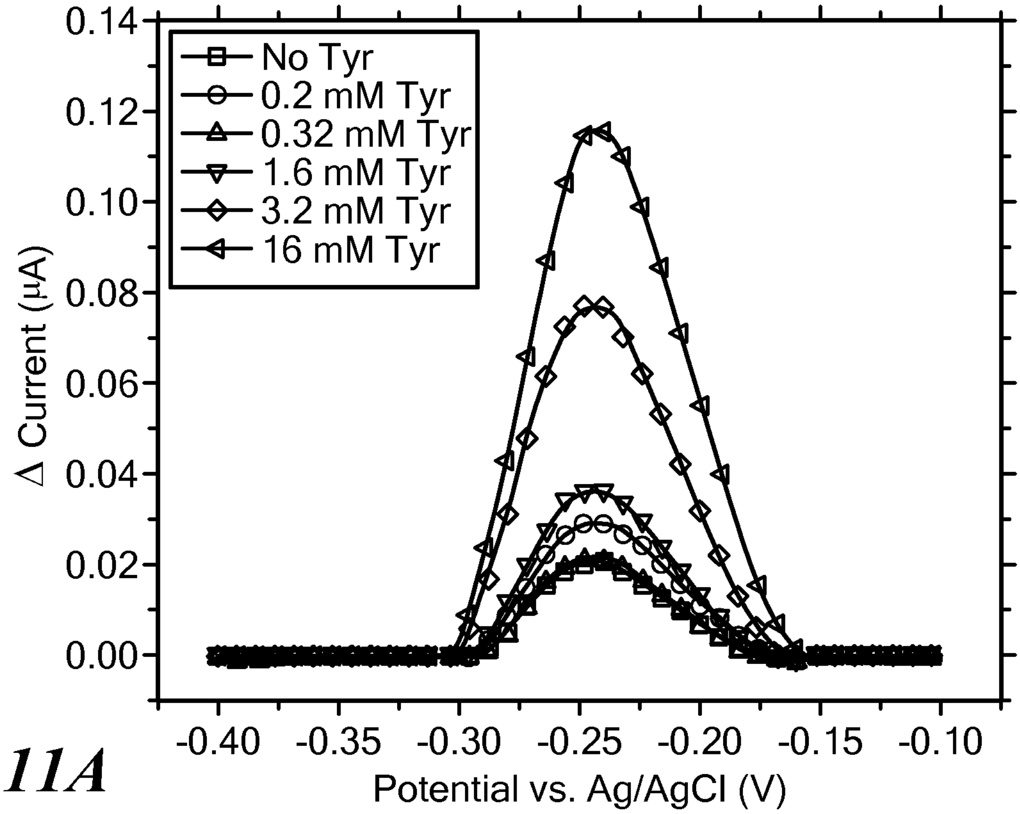


*FIG. 11A*

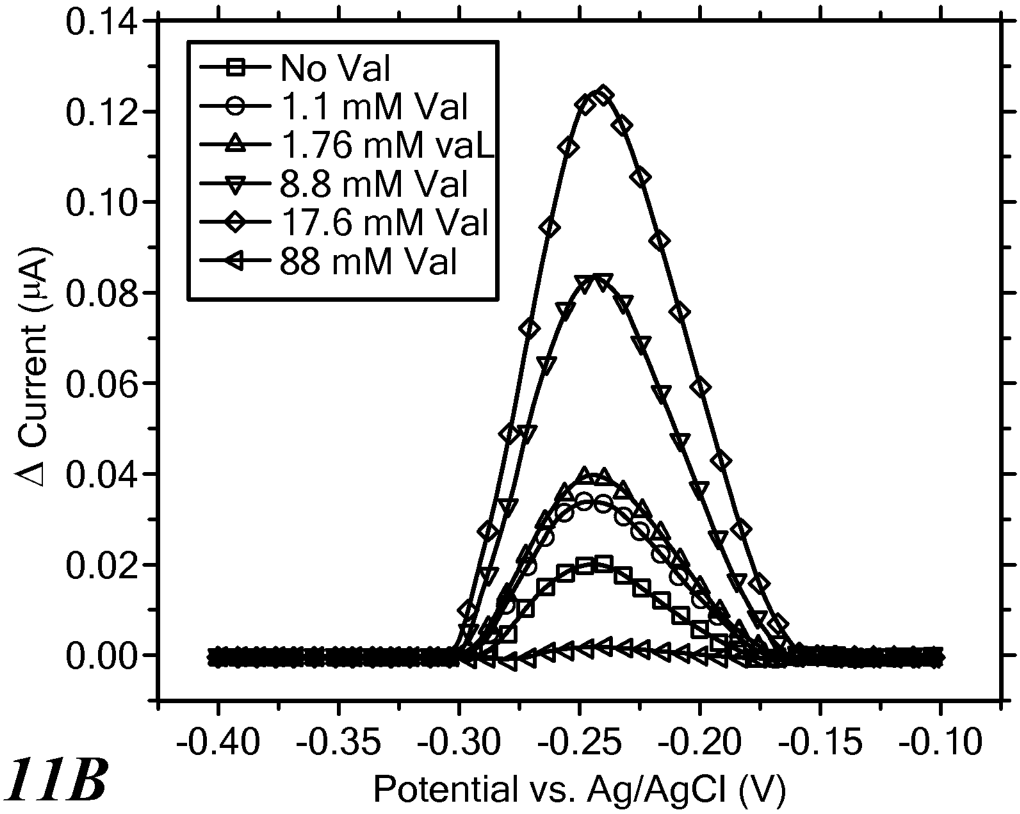


*FIG. 11B*

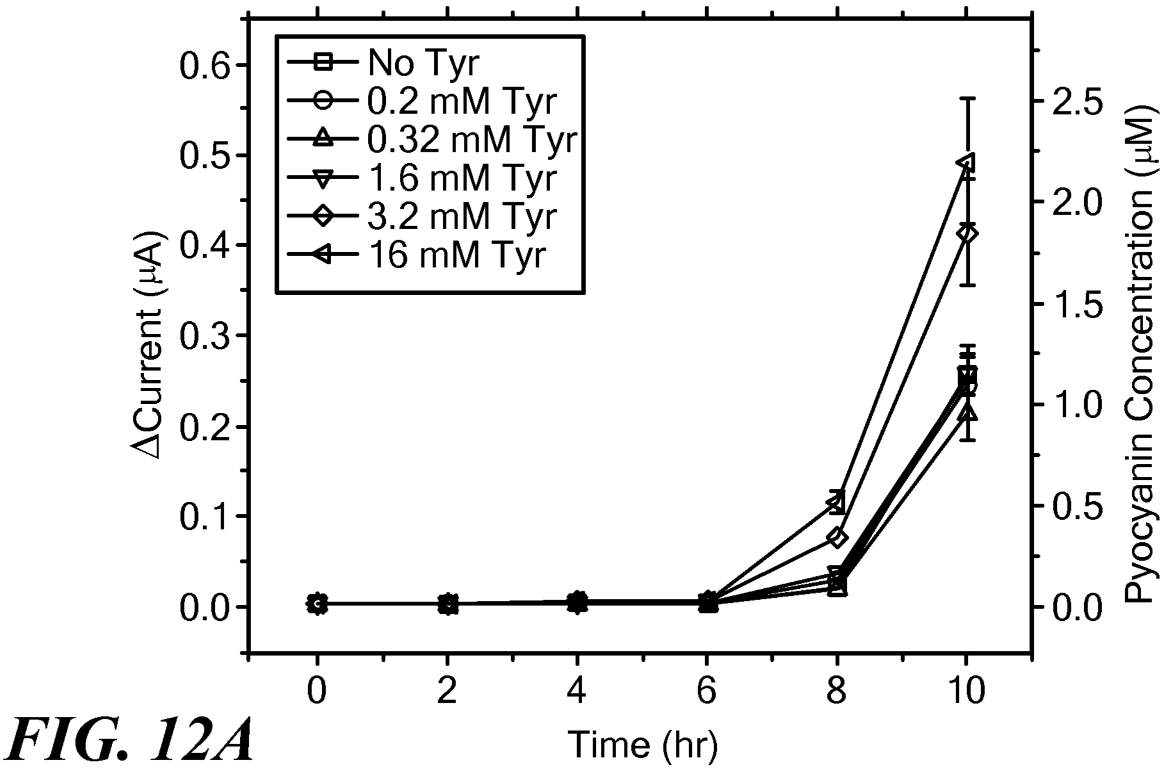


*FIG. 12A*

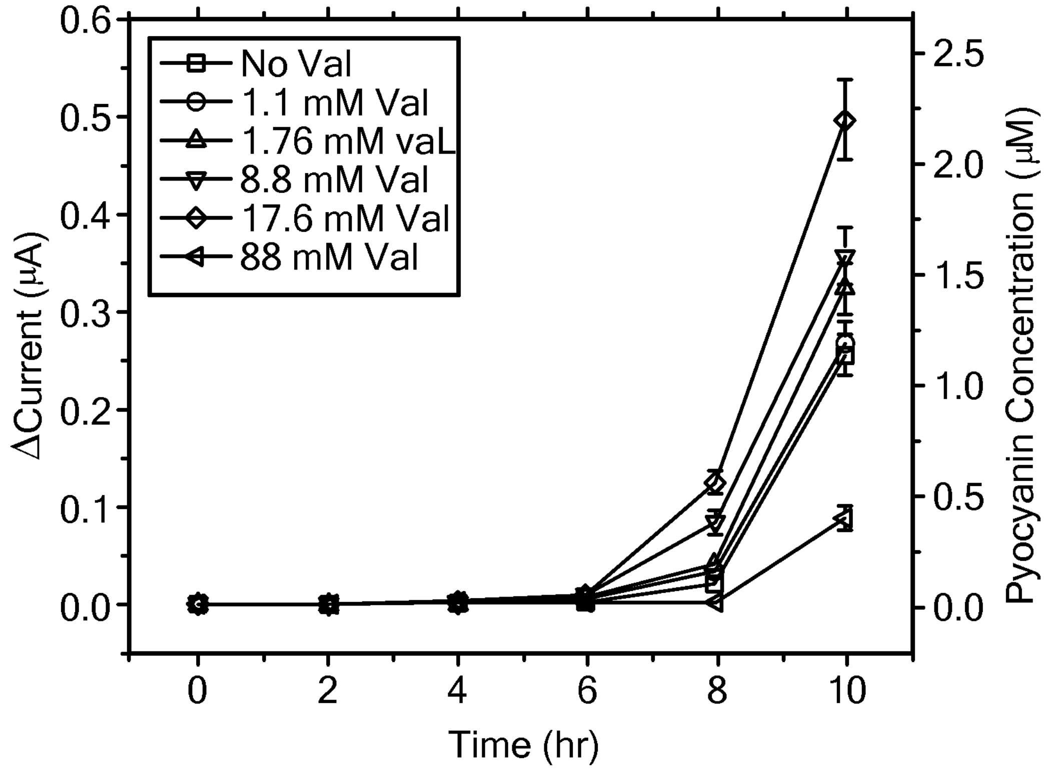


*FIG. 12B*

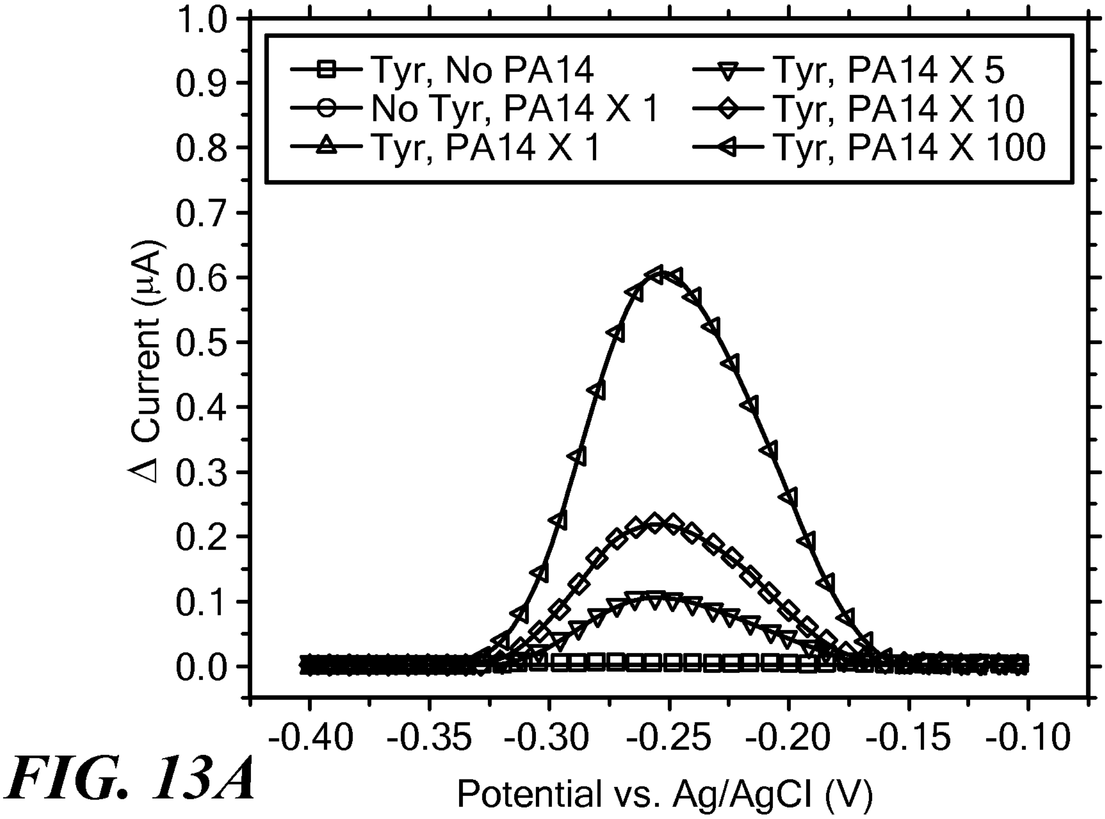


*FIG. 13A*

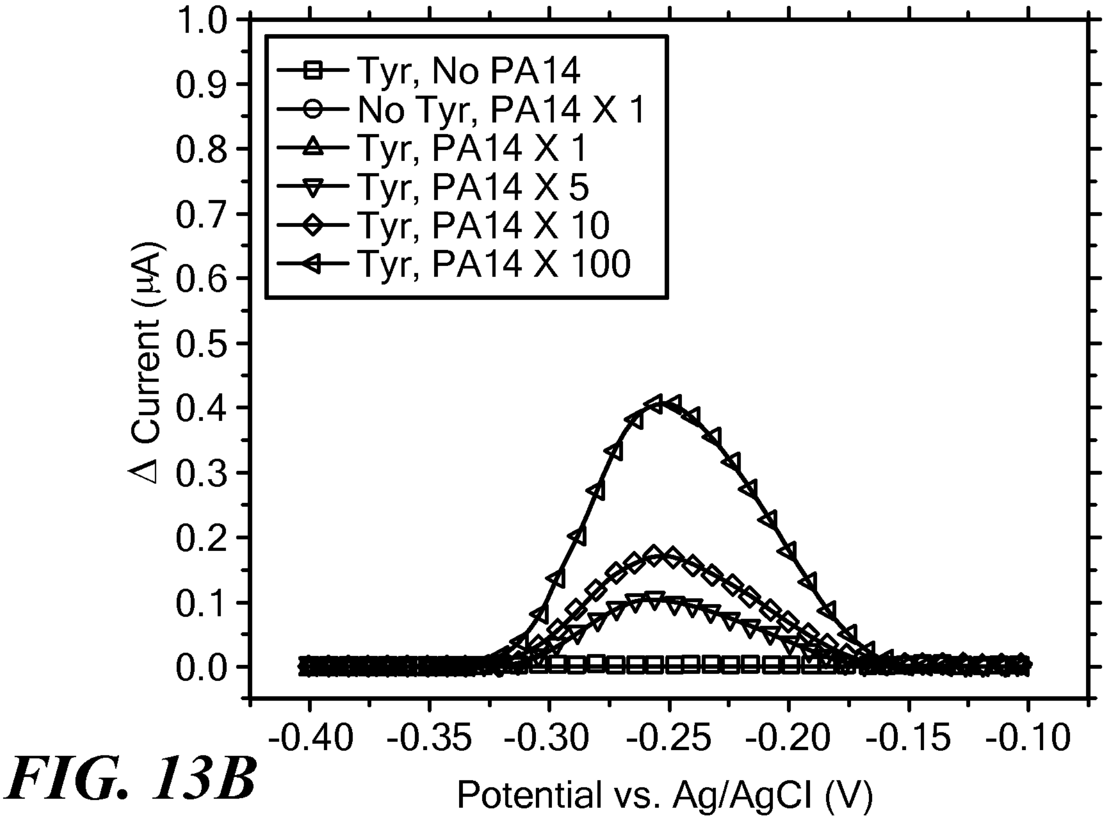


*FIG. 13B*

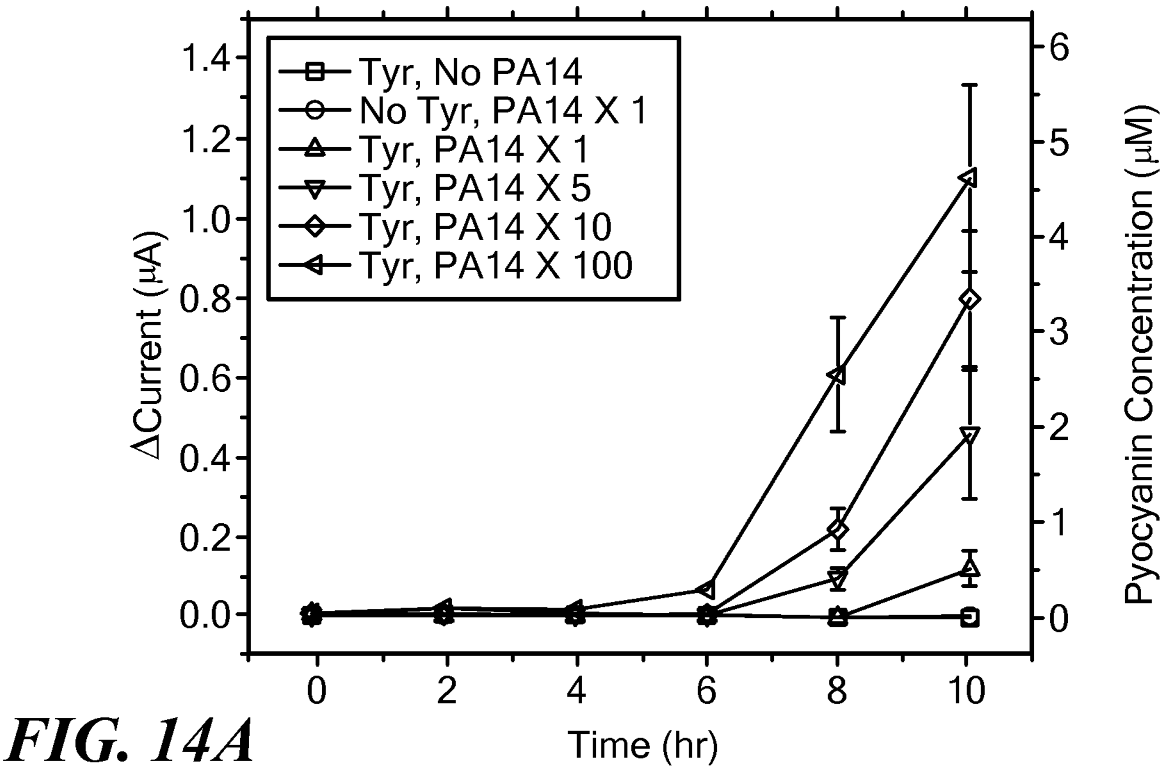


FIG. 14A

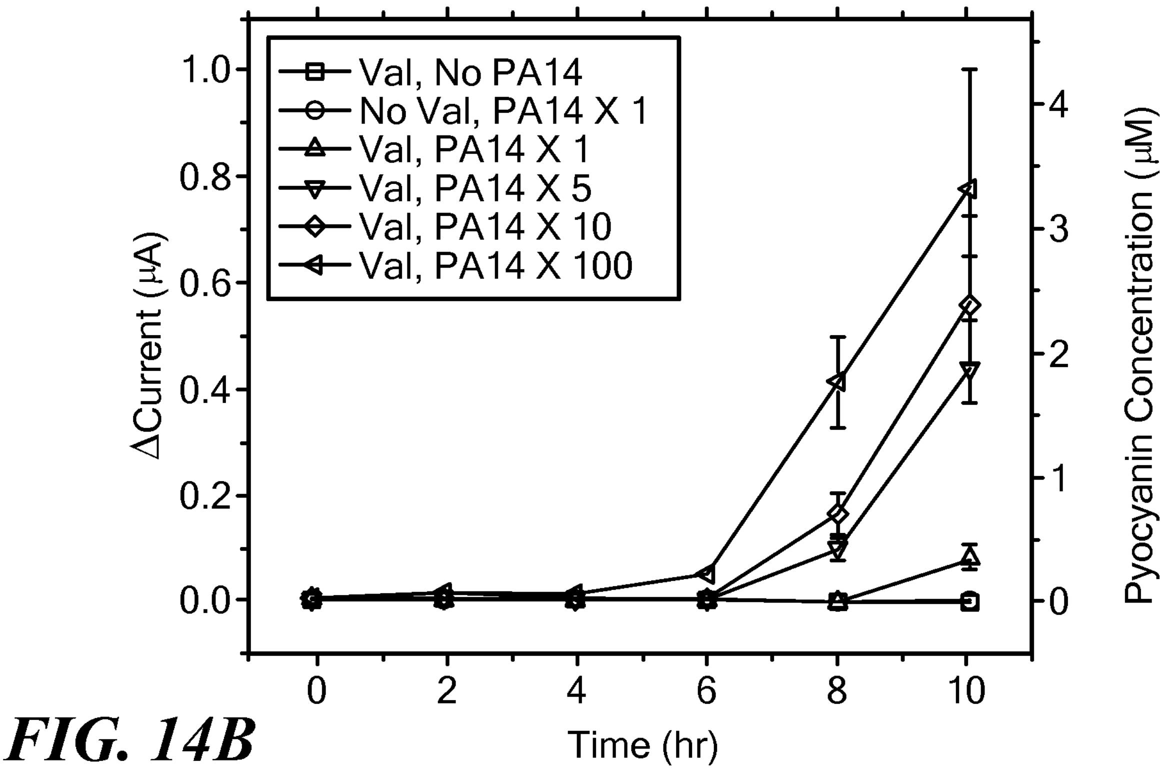


FIG. 14B

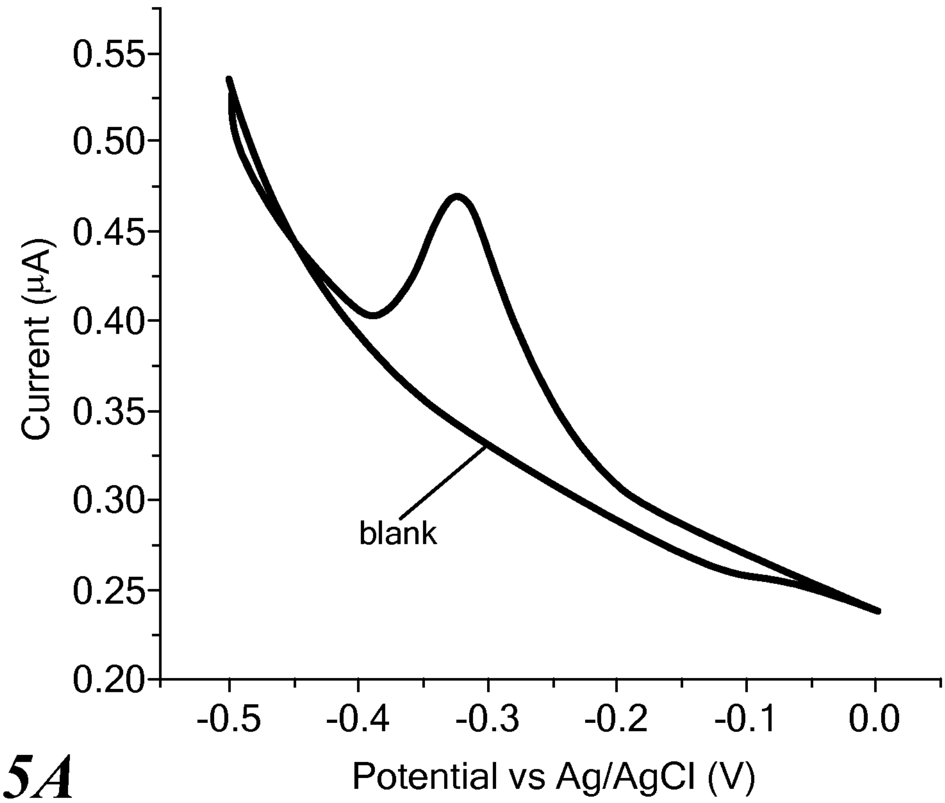


*FIG. 15A*

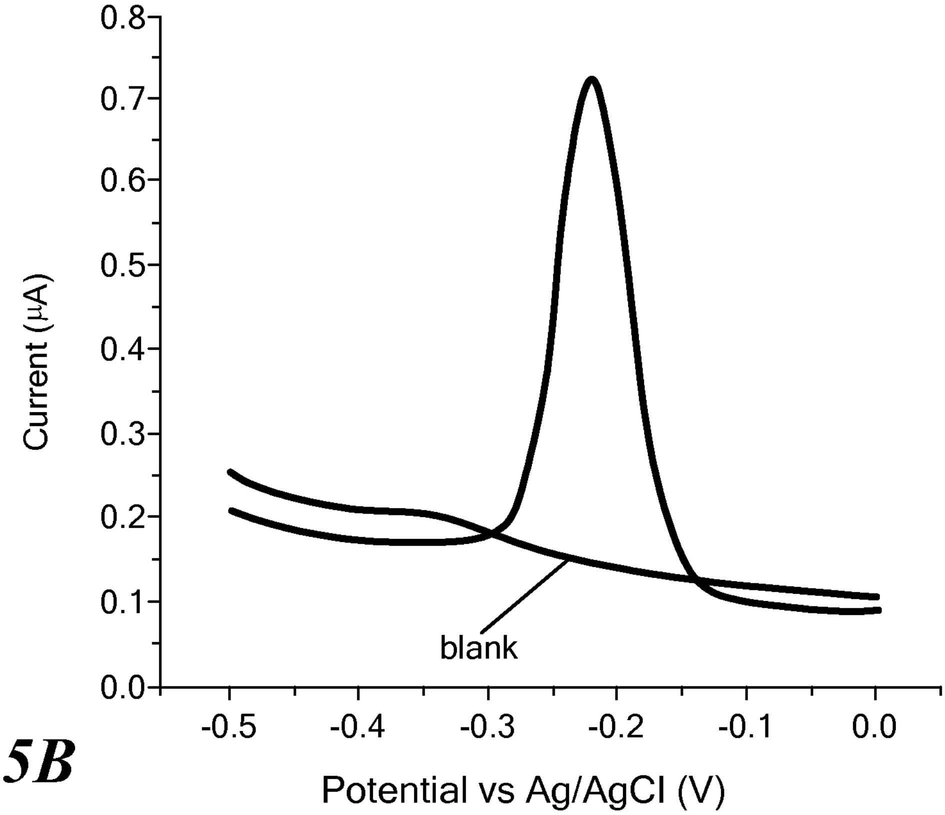


*FIG. 15B*

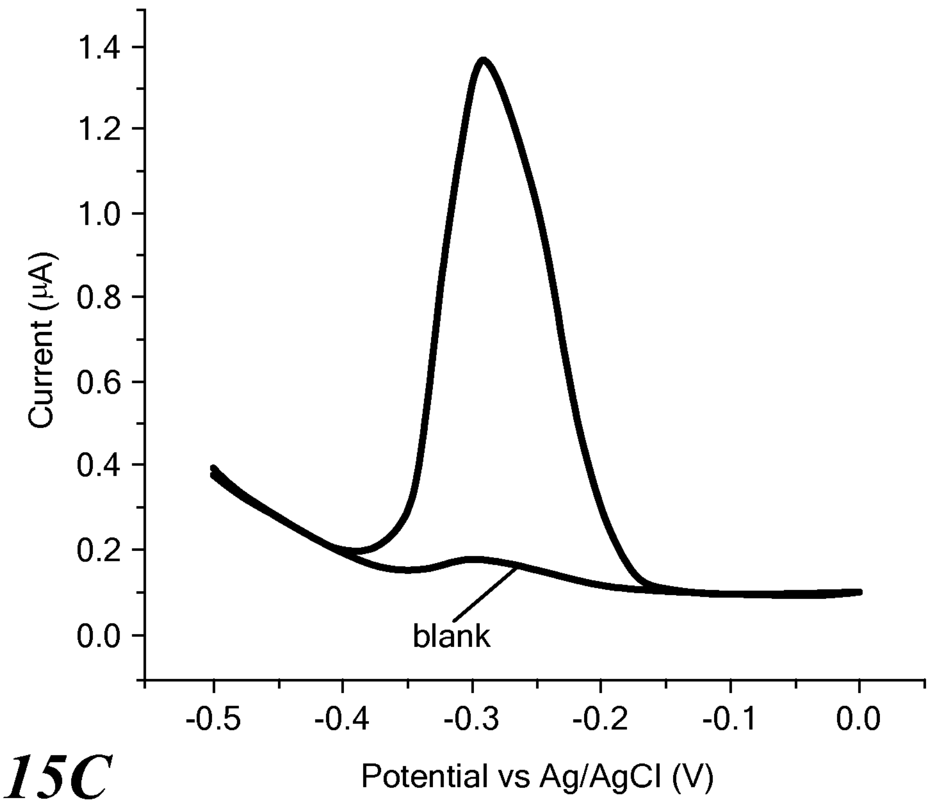


FIG. 15C

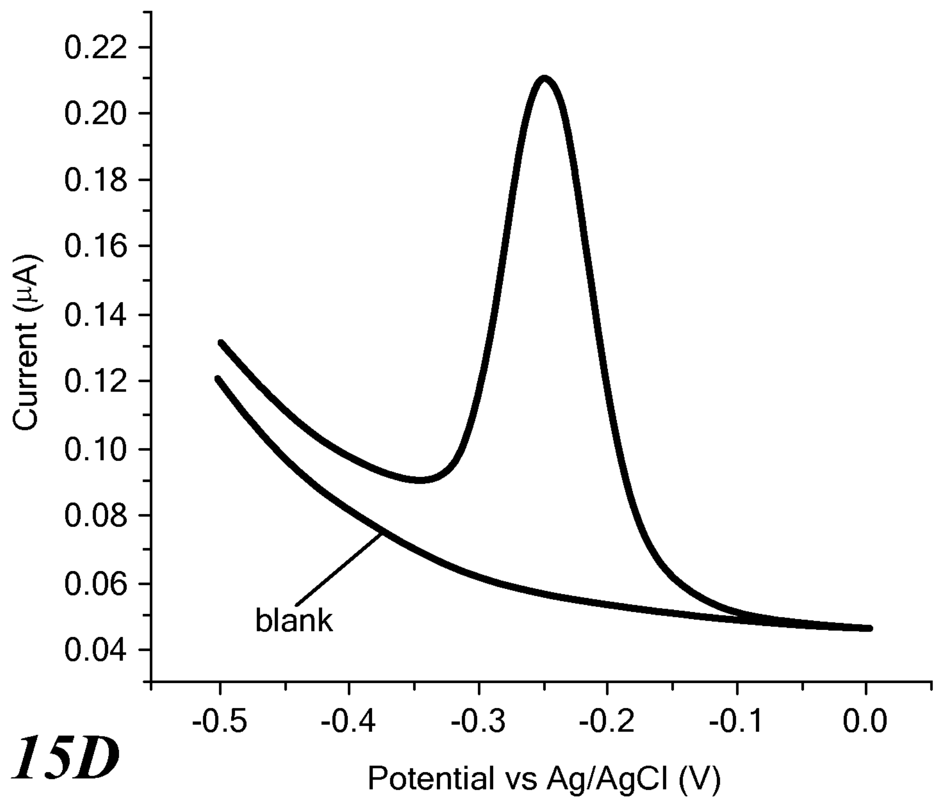


FIG. 15D

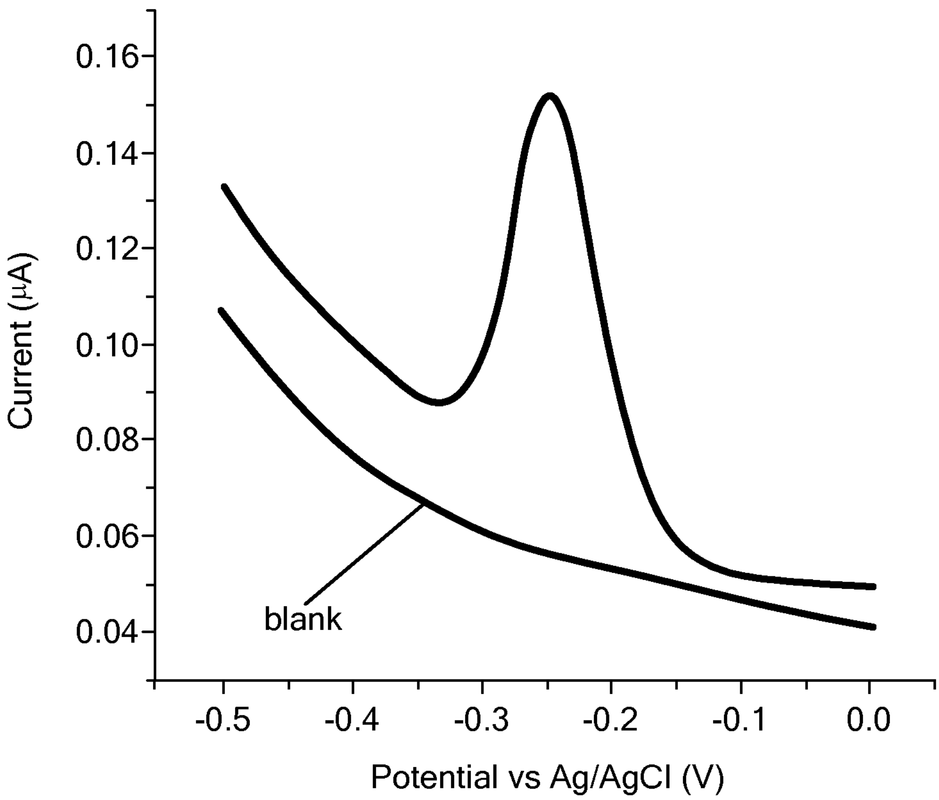
0.16
0.14
0.12
0.10
0.08
0.06
0.04
Current (μA)
blank
-0.5
-0.4
-0.3
-0.2
-0.1
0.0
Potential vs Ag/AgCl (V)

*FIG. 15E*

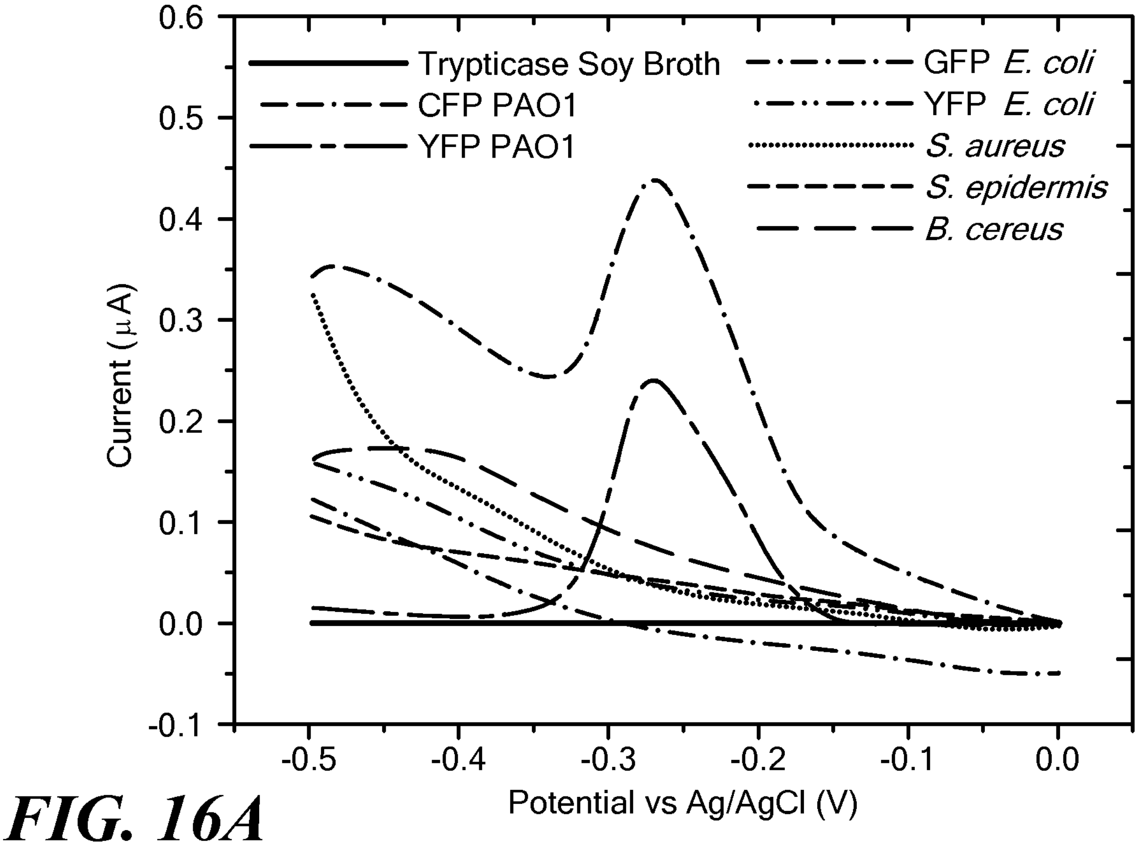


*FIG. 16A*

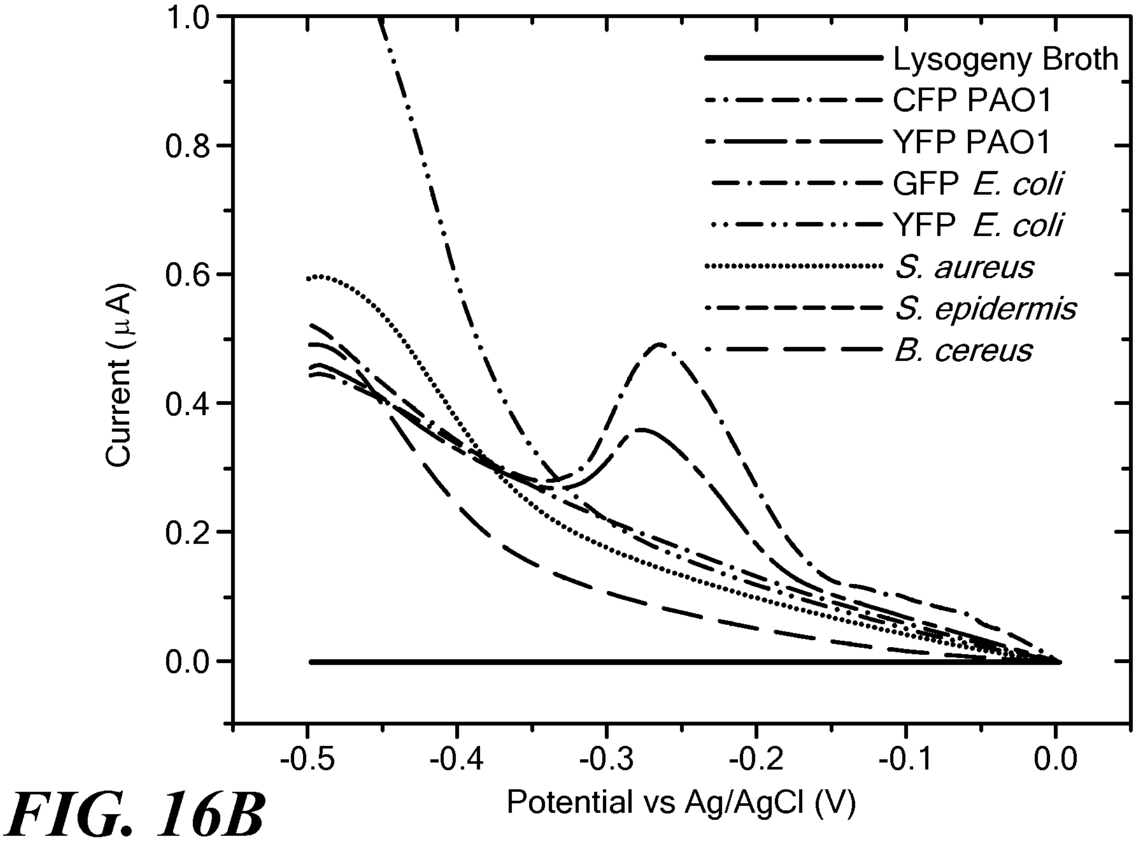


*FIG. 16B*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014015333 A, Goluch, Edgar, D., and Webster, T.


### Non-patent literature cited in the description


- **H. FAZELI ; R. AKBARI ; S. MOGHIM ; T. NARIMANI ; M. R. ARABESTANI ; A. R. GHODDOUSI.** *J. Res. Med. Sci.,* 2012, vol. 17, 332-337
- **V. H. TAM ; K. T. CHANG ; K. ABDELRAOUF ; C. G. BRIOSO ; M. AMEKA ; L. A. MCCASKEY ; J. S. WESTON ; J. P. CAEIRO ; K. W. GAREY.** *Antimicrob. Agents Chemother.,* 2010, vol. 54, 1160-1164
- **T. W. LEE ; K. G. BROWNLEE ; M. DENTON ; J. M. LITTLEWOOD ; S. P. CONWAY.** *Pediatr. Pulmonol.,* 2004, vol. 37, 104-110
- **J. B. LYCZAK ; C. L. CANNON ; G. B. PIER.** *Clin. Microbiol. Rev.,* 2002, vol. 15, 194-222
- **F. RATJEN ; G. DORING.** *Lancet,* 2003, vol. 361, 681-689
- **J. B. LYCZAK ; C. L. CANNON ; G. B. PIER.** *Microbes Infect.,* 2000, vol. 2, 1051-1060
- **E. KIPNIS ; T. SAWA ; J. WIENER-KRONISH.** *Med. Mal. Infect.,* 2006, vol. 36, 78-91
- **G. W. LAU ; D. J. HASSETT ; H. RAN ; F. KONG.** *Trends Mol. Med.,* 2004, vol. 10, 599-606
- **L. E. DIETRICH ; A. PRICE-WHELAN ; A. PETERSEN ; M. WHITELEY ; D. K. NEWMAN.** *Mol. Microbiol.,* 2006, vol. 61, 1308-1321
- **T. A. WEBSTER ; E. D. GOLUCH.** *Lab Chip,* 2012, vol. 12, 5195-5201
- **V. B. WANG ; S. L. CHUA ; B. CAO ; T. SEVIOUR ; V. J. NESATYY ; E. MARSILI ; S. KJELLEBERG ; M. GIVSKOV ; T. TOLKER-NIELSEN ; H. SONG.** *PLoS One,* 2013, vol. 8, e63129
- **D. SHARP ; P. GLADSTONE ; R. B. SMITH ; S. FORSYTHE ; J. DAVIS.** *Bioelectrochemistry,* 2010, vol. 77, 114-119
- **T. A. WEBSTER ; H. J. SISMAET ; E. D. GOLUCH.** *Nano LIFE,* 2013, vol. 03, 1340011
- **T. A. WEBSTER ; H. J. SISMAET ; J. L. CONTE ; I. P. CHAN ; E. D. GOLUCH.** *Biosens. Bioelectron.,* 2014, vol. 60, 265-270
- **S. P. BERNIER ; D. G. HA ; W. KHAN ; J. H. MERRITT ; G. A. O'TOOLE.** *Res. Microbiol.,* 2011, vol. 162, 680-688
- **E. HELLINGER.** *J. Gen. Microbiol.,* 1951, vol. 5, 633-639
- **N. GROSSOWICZ ; P. HAYAT ; Y. S. HALPERN.** *J. Gen. Microbiol.,* 1957, vol. 16, 576-583
- **W. HE ; C. LI ; C. D. LU.** *J. Bacteriol.,* 2011, vol. 193, 2107-2115
- **S. R. THOMAS ; A. RAY ; M. E. HODSON ; T. L. PITT.** *Thorax,* 2000, vol. 55, 795-797
- **L. S. PIERSON, 3RD ; E. A. PIERSON.** *Appl. Microbiol. Biotechnol.,* 2010, vol. 86, 1659-1670
- **R. C. HIDER ; X. KONG.** *Nat. Prod. Rep.,* 2010, vol. 27, 637-657
- **LACHER et al.** *Electrophoresis,* 2001, vol. 22, 2526-2536
- **DAM et al.** *Analyst,* 2007, vol. 132, 365-370
- **PIHEL et al.** *Anal Chem,* 1995, vol. 67, 4514-4521
- **STRAVER et al.** *Lab Chip,* vol. 12, 1548-1553
- **GOLUCH et al.** *Anal Bioanal Chem,* 2009, vol. 394, 447-456
- **ZEVENBERGEN et al.** *Nano Lett,* 2007, vol. 7, 384-388
- **ZEVENBERGEN et al.** *Nano Lett,* 2011, vol. 11, 2881-2886
- **WOLFRUM et al.** *Anal Chem,* 2008, vol. 80, 972-977
- **WEBSTER ; GOLUCH.** *Lab Chip,* 2012, vol. 12, 5195-5201
- **SHARP et al.** *Bioelectrochem,* 2010, vol. 77, 114-119
- **G. C. PALMER ; K. L. PALMER ; P. A. JORTH ; M. WHITELEY.** *J. Bacteriol.,* 2010, vol. 192, 2722-2728
- **D. J. MUSK ; D. A. BANKO ; P. J. HERGENROTHER.** *Chem. Biol.,* 2005, vol. 12, 789-796
- **CZERWINSKI ; MARASSI.** *J Electroanaly Chem Interfacial Electrochem,* 1992, vol. 322, 373-381
- **CZERWINSKI et al.** *J Electroanal Chem Interfacial Electrochem,* 1991, vol. 316, 211-221

- **CZERWINSKI et al.** *J Electroanal Chem Interfacial Electrochem,* 1995, vol. 386, 207-211
- **ODIJK et al.** *Lab Chip,* 2009, vol. 9, 1687-1693
- **ZENG et al.** *Nano Lett,* 2011, vol. 11, 262-268